# EUROPEAN PATENT SPECIFICATION

(11) **EP 1 558 935 B1**
(45) Date of publication and mention of the grant of the patent: **09.07.2008**
(21) Application number: 03809236.7
(22) Date of filing: 27.10.2003
(51) Int. Cl.: G01R 1/00

(54) **CONTROLLED-RELEASE COMPOSITIONS**
ZUBEREITUNGEN MIT KONTROLLIERTER FREISETZUNG
COMPOSITIONS A LIBERATION CONTROLEE

(30) Priority: 25.10.2002 US 509062 P; 10.10.2003 US 510000 P
(43) Date of publication of application: 03.08.2005
(73) Proprietor: Labopharm Inc., Laval, Quebec H7V 4B4 (CA); Labopharm Europe Limited, Dublin 3 (IE); Labopharm (Barbados) Limited, Belleville Saint Michael (BB)
(72) Inventor: LENAERTS, Vincent, Beaconsfield, Québec H9W 5E2 (CA); OUADJI-NJIKI, Patricia Laure, Montreal, Québec H2C 3A5 (CA); BACON, Jonathan, Montreal, Québec H1X 3A2 (CA); Ouzerourou, Rachid, Montreal, Québec H1K 1L8 (CA); GERVAIS, Sonia, Laval, Québec H7L 5E8 (CA); RAHMOUNI, Miloud, Pierrefonds (Québec) H9J 3P9 (CA); SMITH, Damon, St-Laurent, Québec H4R 3A5 (CA)
(74) Representative: Casalonga, Axel
(86) International application number: PCT/CA2003/001637
(87) International publication number: WO 2004/038428

(56) References cited:
- EP-A- 0 624 366
- EP-A- 1 138 320
- WO-A-99/01111
- WO-A-02/060415
- US-A- 5 776 492
- US-B1- 6 419 957
- BHAVESH BODALIA ET AL.: "A Comparison of the Pharmacokinetics, Clinical Efficacy, and Tolerability of Once-Daily Tramadol Tablets with Normal Release Tramadol Capsules" JOURNAL OF PAIN AND SYMTOM MANAGEMENT, vol. 25, no. 2, February 2003 (2003-02), pages 142-149, XP001180925 cited in the application
- L. ADLER ET AL.: "A Comparison of Once-Daily Tramadol with Normal ReleaseTramadol in the Treatment of Pain in Osteoarthritis" THE JOURNAL OF RHEUMATOLOGY, vol. 29, no. 10, 2002, pages 2196-2199, XP009029330 cited in the application
- VISAVARUNGROJ N ET AL: "CROSSLINKED STARCH AS A DISINTEGRATING AGENT" INTERNATIONAL JOURNAL OF PHARMACEUTICS, AMSTERDAM, NL, vol. 62, no. 2/3, 1990, pages 125-131, XP000933913 ISSN: 0378-5173

## Description

### FIELD OF THE INVENTION

This invention relates to a solid dosage formulation in which an active ingredient is released over a sustained period.

### BACKGROUND OF THE INVENTION

An important factor influencing the rate of absorption of an active agent administered as a tablet or other solid dosage formulation, and thus the efficacy and safety of the formulation, is the rate of dissolution of the dosage form in the body fluids of a human or animal.

The ability of components of the formulation to influence the rate of release of the active agent(s) thus constitutes the basis for the so-called controlled-release, extended-release, sustained-release or prolonged-action pharmaceutical preparations that are designed to produce slow, uniform release and absorption of the active agent over a period of hours, days, weeks, or months. Advantages of controlled-release formulations include a reduction in the required frequency of administration of the drug as compared to immediate-release dosage forms, often resulting in improved patient compliance; maintenance of a relatively stable concentration of the drug in the body leading to a sustained therapeutic effect over a set period of time; and decreased incidence and intensity of undesired side effects of the active agent resulting from a reduction of the high plasma concentrations that often occur after administration of immediate-release dosage forms.

Many materials have been proposed and developed as matrices for controlled release of active agents, i.e. drugs, pro-drugs, etc. These include polymeric materials such as polyvinyl chloride, polyethylene amides, ethyl cellulose, silicone and poly (hydroxymethyl methacrylate). See, for example, U.S. Patent No. 3,087,860 to Endicott et al.*;* U.S. Patent No. 2,987,445 to Levesque et al.*;* Salomon et al., Pharm. Acta Helv., 55, 174-182 (1980); Korsmeyer, Diffusion Controlled Systems: Hydrogels, Chap. 2, pp 15-37 in Polymers for Controlled Drug Delivery, Ed Tarcha, CRC Press, Boca Raton, Fla. USA (1991); and Buri et al., Pharm. Acta. Helv. 55, 189-197 (1980).

High amylose starch has also been used for controlled-release purposes, and, in particular, recent advances have been made using cross-linked high amylose starch. For example, U.S. Patent No. 5,456,921 (Mateescu et al.), which issued October 10, 1995, U.S. Patent No. 5,616,343 (Cartilier et al.), which issued April 1, 1997, U.S. Patent No. 6,284,273 (Lenaerts et al.), which issued September 4, 2001, U.S. Patent No. 6,419,957 (Lenaerts et al.), which issued July 16, 2002, and U.S. Patent No. 6,607,748 (Lenaerts et al.), which issued August 19, 2003, describe solid controlled release oral pharmaceutical dosage units in the form of tablets comprising a dry powder of a pharmaceutical product and a dry powder of cross-linked high amylose starch in which the cross-linked high amylose starch includes a mixture of about 10-60% by weight of amylopectin and about 40-90% amylose.

Further examples of controlled-release materials include Kollidon^{™} SR marketed by BASF (Germany), this material being a physical mixture of polyvinyl acetate (PVA) and polyvinylpyrrolidone (povidone), reportedly made up of about 80% PVA and 19% povidone, and approximately 0.8% sodium dodecylsulfate and about 0.2% silica as stabilizer. BASF Technical Information (July 2001) discloses that Kollidon^{™} SR can be used in the preparation of sustained release matrix dosage forms including tablets, pellets and granules, and that different technologies such as direct compression, roller compaction, wet granulation and extrusion may be employed in the manufacture of pharmaceutical formulations. A number of patent publications provide further information on PVA-povidone mixtures: U.S. Patent Publication No. 2001/0038852 (Kolter et al.) published November 8, 2001; U.S. Patent Publication No. 2002/0012701 (Kolter et al.) published January 31, 2002, and U.S. Patent Publication No. 2003/0021846 (Kolter et al.) published January 30, 2003.

Extended and controlled release formulations relating to tramadol have been suggested, examples being described in: U.S. Patent Publication No. 2003/0143270, (Deboeck et al.) published July 31, 2003; U.S. Patent No. 6,254,887 (Miller et al.) issued July 3, 2001; U.S. Patent Publication No. 2001/0036477 (Miller et al.) published November 1, 2001; U.S. Patent No: 6,326,027 (Miller et al.) issued December 4, 2001, WO 03/080031 (CILAG AG et al.) published October 2, 2003. Articles have been published In which comparative data between "once-daily" tramadol formulations and immediate release tramadol formulations are presented: Adler et al., "A Comparison of Once-Daily Tramadol with Normal Release Tramadol in the Treatment of Pain in Osteoarthritis," The Journal of Rheumatology (2002) 29(10): 2195-2199; and Bodalia et al., "A Comparison of the Pharmacokinetics, Clinical Efficacy, and Tolerability of Once-Daily Tramadol Tablets with Normal Release Tramadol Capsules," Journal of Pain and Symptom Management (2003) 25(2): 142-149.

Citation or identification of any reference in this specification is not intended to be construed as an admission that such reference is available as prior art to the present invention.

### SUMMARY OF THE INVENTION

The present invention relates to a solid dosage formulation comprising: a core comprising a pharmacological agent dispersed in a first controlled release matrix and a coat formed over the core comprising a pharmacological agent dispersed in a second controlled release matrix,
wherein
the first controlled release matrix comprises cross-linked high amylose starch and/or the second controlled release matrix comprises a physical mixture of polyvinyl acetate and polyvinylpyrrolidone and
the initial rate of release of the agent for the second controlled release matrix is a least twice as fast as the rate of release of the agent from the first controlled release matrix when measured separately for each matrix material under USP Type I conditions with 50 mM sodium phosphate buffer and pH 6.8 and 100 rpm .

The formulation provides for controlled-release of a pharmacological agent. The formulation includes a core having a pharmacological agent dispersed in a first controlled-release matrix comprising cross-linked high amylose starch, from which matrix release of the agent is relatively slow. There is a coat formed over the core and the coat includes the agent dispersed in a second controlled-release matrix from which release of the agent is relatively fast.

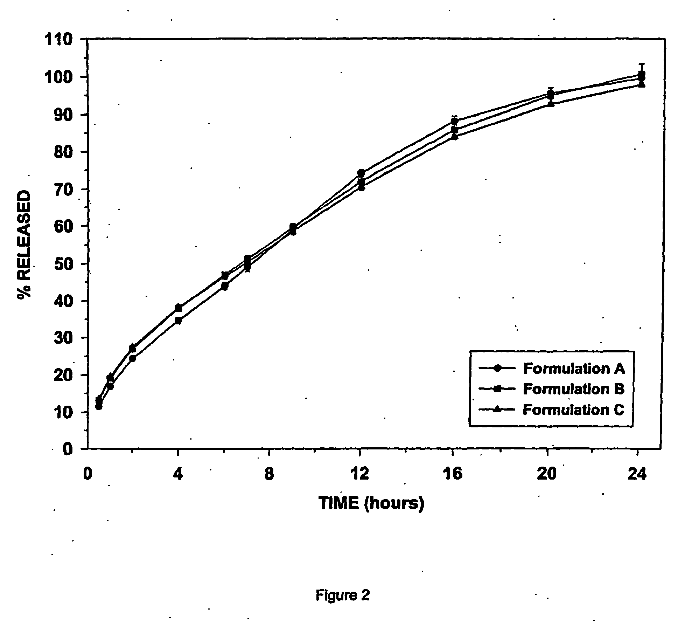

In the context of this invention, "relatively fast" means at least twice as fast when the initial rate of release of an agent is measured under the same conditions separately for each matrix material. To make such a measurement, one makes a formulation having the agent of the core and the agent of the coat differentially labeled from each other. In the case of tramadol, for example, the tramadol of the core could be labeled with ¹⁵N and the tramadol of the coat could be labeled with ¹³C. There are many ways known to a skilled person for differentially labeling such a compound so that its diffusion from the formulation can be traced without significantly affecting its rate of diffusion. A skilled person could estimate such relative rates to a reasonable approximation, provided the rates are sufficiently different from each other, from the biphasic behavior observed for release of the agent from a single formulation, e.g., from the rates at t=0, and t=12 hr of Figure 2. Typically, the measurement would be made under the conditions set forth in connection with Figure 2.

In another broad embodiment, the invention is a solid dosage formulation having a core with a pharmacological agent in a first controlled-release matrix. There is a coat formed over the core having the pharmacological agent in a second controlled-release matrix. The second controlled-release matrix is a physical mixture of polyvinyl acetate and polyvinylpyrrolidone, and release of the agent from the matrix of the core is relatively slow with respect to release of the agent from the matrix of the coat. Relatively slow means no more than half as fast when the initial rate of release of an agent is measured under the same conditions separately for each matrix material, the measurement being determined as described above in connection with the determination of relatively fast.

The agent in the core and coat may, in either embodiment, be the same or different. In a preferred embodiment, the formulation includes a single agent that is tramadol.

In a preferred aspect of the invention, the coat and core comprise relative amounts of the agent such that release of the agent from the formulation is biphasic.

Preferably, the agent is soluble in water, and the first matrix is relatively hydrophilic relative to the second matrix.

Many agents are capable of forming ionic salts, and this is often the preferred form of the agent for incorporation into a formulation of the invention. Preferred agents contain at least one amino group, and these are conveniently incorporated in the form of, for example a hydrochloride salt.

Preferably, the rate of release of the agent from the coat is at least twice the rate of release of the agent from the core. Other relative rates are possible: the rate of release of the agent from the coat can be at least three times the rate of release of the agent from the core; the rate of release of the agent from the coat can be up to fifteen times the rate of release of the agent from the core; the rate of release of the agent from the coat can be up to twelve times the rate of release of the agent from the core; the rate of release of the agent from the coat can be up to ten times the rate of release of the agent from the core; the rate of release of the agent from the coat can be up to eight times the rate of release of the agent from the core; the rate of release of the agent from the coat can be up to six times the rate of release of the agent from the core; or the rate of release of the agent from the coat can be about four times the rate of release of the agent from the core. In other embodiments, biphasic release behavior is observed, and the rate of release of the agent from the coat is between three and nine times the rate of release of the agent from the core, more preferably the rate of release of the agent from the coat is between four and eight times the rate of release of the agent from the core, more preferably the rate of release of the agent from the coat is between five and seven times the rate of release of the agent from the core.

In certain embodiments, between 10% and 30% per hour of the agent is released between 0 and 2 hours when tested *in vitro* using a USP Type I apparatus in 50 mM phosphate, pH 6.8, and stirring between 50 and 150 rpm.

In certain embodiments, between 10% and 40% of the agent is released from the formulation between 0 and about 2 hours of measurement, between about 30% and 60% of the agent is released from the formulation between 2 and about 7 hours of the measurement, between about 50% and 80% of the agent is released from the formulation between 7 and about 12 hours of measurement, and between about 80% and 100% of the agent is released from the formulation after about 20 hours of measurement.

A preferred active agent of both the core and the coat is an analgesic, specifically, the active agent can be tramadol.

An agent of the formulation of the invention is preferred to be soluble in water at least to the extent of 1 g/L, or more than 10 g/L or more than 100 g/L, or more than 500 g/L, or more than 1000 g/L, or more than 2000 g/L.

In certain embodiments, the formulation of the invention is generated to have the ratio of the core to the coat (w/w) between about 1 and about 0.1, or between about 0.9 and about 0.2, or between about 0.8 and about 0.2, or between about 0.7 and about 0.2, or between about 0.5 and about 0.2, or between about 0.4 and about 0.2, or about 0.35. In this context, it is the total weight of the core and the total weight of the coat that would be considered when determining the weight ratio.

In certain embodiments, the ratio of the agent in core to the agent in the coat (w/w) is between about 0.1 and about 10, or between about 0.1 and about 8, or between about 0.2 and about 7, or between about 0.3 and about 6, or between about 0.4 and about 5, or between about 0.5 and about 4, or between about 0.6 and about 3, or between about 0.6 and about 2, or between about 0.6 and about 1.5, or between about 0.6 and about 1.3, or between about 0.7 and about 1, or between about 0.7 and about 0.9 or about 0.8.

In particular embodiments of the invention, a formulation is one in which the core is between about 10% and about 90% by weight agent, or between about 20% and about 80% by weight agent, or between about 30% and about 70% by weight agent, or between about 40% and about 60% by weight agent, or about 50% by weight agent.

In particular embodiments, a formulation of the invention is one in which the coat is between about 5% and about 90% by weight agent, or between about 5% and about 80% by weight agent, or between about 10% and about 70% by weight agent, or between about 10% and about 60% by weight agent, or between about 15% and about 50% by weight agent, or between about 15% and about 45% by weight agent, or between about 15% and about 40% by weight agent, or between about 20% and about 35% by weight agent, or between about 20% and about 30% by weight agent.

According to certain aspects of the invention, the formulation is such that the ratio of the matrix of the coat to the agent of the coat (w/w) is between about 0.1 and about 10, or between about 0.2 and about 9, or between about 0.2 and about 8, or between about 0.3 and about 7, or between about 0.4 and about 6, or between about 0.5 and about 5, or between about 0.6 and about 4, or between about 0.7 and about 4 or between about 1 and about 4, or between about 1 and about 3 and about 1.5 and about 2.5.

According to certain aspects, the formulation is such that the ratio of the matrix of the core to the agent of the core (w/w) is between about 0.1 and about 10, or between about 0.2 and about 9, or between about 0.3 and about 7, or between about 0.4 and about 6, or between about 0.5 and about 5, or between about 0.5 and about 4, or between about 0.5 and about 3, or between about 0.6 and about 3, or between about 0.7 and about 2 or between about 0.8 and about 1.5, or between about 0.9 and about 1.5, or between about 0.9 and about 1.3, or about 1, or is about 0.55.

Preferably, the agent is a single agent soluble in water at room temperature (about 21°C) to the extent of at least 0.5 gm per mL.

In certain aspects, each agent of the formulation contains an acid group, a base group or both an acid group and a base group, and each agent is present in the form of a salt of such group. Preferably, the agent contains an ionizable group and said group is at least 90% ionized in gastric juices (0.1 M HCl).

Agents of a formulation of the invention can be any one or more of the following: isonicotinic acid hydrazide, sodium salicylate, pseudoephedrine hydrochloride, pseudoephedrine sulfate, acetaminophen or diclofenac sodium, verapamil, glipizide, nifedipine, felodipine, betahistine, albuterol, acrivastine, omeprazole, misoprostol, tramadol, oxybutynin, trimebutine, ciprofloxacin, and salts thereof. In addition, the pharmaceutical agent can be an antifungal agent, such as ketoconazole, or an analgesic agent such as acetylsalicylic acid, acetaminophen, paracetamol, ibuprofen, ketoprofen, indomethacin, diflunisal, naproxen, ketorolac, diclofenac, tolmetin, sulindac, phenacetin, piroxicam, mefamanic acid, dextromethorphan, other non-steroidal anti-inflammatory drugs including salicylates, pharmaceutically acceptable salts thereof or mixtures thereof.

Preferably, a formulation of the invention is prepared by compression. Typically, the core is formed, by compression, and then the coat is prepared by being compressed onto the pre-formed core.

In a preferred aspect, the coat is made up of an admixture of polyvinyl acetate, polyvinylpyrrolidone. The ratio of polyvinyl acetate and polyvinylpyrrolidone in the coat (w/w) is usually between about 6:4 and 9:1, or 7:3 and 9:2, or it is about 8:2.

The coat often includes a binding agent, a preferred binding agent being xanthan gum.

The formulation can be a tablet, and a preferred cross-linked high amylose starch is a chemically-modified, cross-linked high amylose starch prepared by a method comprising:
(a) cross-linking high amylose starch, followed by
(b) chemically modifying the cross-linked high amylose starch, followed by
(c) gelatinization, and
(d) drying to obtain a powder of said controlled release excipient;
wherein said cross-linked high amylose starch is characterized in that upon solubilization in 90% DMSO at 80°C. for about three days and gel permeation chromatography, the height of the peak corresponding to amylose in said cross-linked high amylose starch is at least 90% of that of the peak corresponding to amylose in said high amylose starch prior to (a).

Another process for obtaining a cross-linked high amylose starch for formulations of this invention includes:
(a) cross-linking high amylose starch thereby forming a reaction medium containing a reaction product consisting of a cross-linked high amylose starch slurry;
(b) subjecting said cross-linked high amylose starch slurry from step (a) to chemical modification at a temperature of about 10 to about 90°C. for about 1 to about 72 hours;
(c) neutralizing said reaction medium obtained in step (b) with an acid, washing the slurry formed and optionally dewatering to form a starch cake or a dry powder,
(d) diluting said slurry or re-slurrifying said starch cake or said dry powder from step (c) with water to form a slurry at a concentration of about 2% to about 40% w/w, adjusting pH to a desired value between about 3 and about 12, and gelatinizing said slurry at a temperature of about 80 to 180°C. for about 1 second to about 120 minutes; and
(e) drying the thermally treated product obtained in step (d) to obtain said controlled release excipient consisting mainly of chemically modified and cross-linked high amylose starch in form of a powder.

Another process for manufacturing, in an aqueous medium, a controlled release excipient consisting primarily of cross-linked high amylose starch is one including
(a) subjecting high amylose starch to chemical modification at a temperature of about 10 to about 90°C. for about 1 to about 72 hours thereby forming a reaction medium containing a chemically modified high amylose slurry,
(b) cross-linking said chemically modified high amylose starch in said slurry obtained in step (a);
(c) neutralizing said slurry obtained in step (b) with an acid, washing the slurry formed and optionally dewatering to form a starch cake or drying to form dry powder,
(d) diluting said slurry, or re-slurrifying said starch cake or said dry powder from step (c) with water to form a slurry at a concentration of about 2% to about 40% w/w, adjusting pH to a desired value between about 3 and about 12, and gelatinizing said slurry at a temperature of about 80 to 180°C. for about 1 second to about 120 minutes; and
(e) drying the thermally treated product obtained in step (d) to obtain said controlled release excipient consisting mainly of chemically modified and cross-linked high amylose starch in form of a powder.

Another process for obtaining a cross-linked high amylose starch for this invention includes:
(a) cross-linking high amylose starch, followed by
(b) chemically modifying the cross-linked high amylose starch, followed by
(c) gelatinization, and
(d) drying to obtain a powder of said controlled release excipient;
wherein said cross-linked high amylose starch is characterized in that upon solubilization in 90% DMSO at 80°C. for about three days and gel permeation chromatography, the height of the peak corresponding to amylose in said cross-linked high amylose starch is at least 90% of that of the peak corresponding to amylose in said high amylose starch prior to (a).

Another process for obtaining a cross-linked high amylose starch for this invention includes:
(a) cross-linking high amylose starch, followed by
(b) chemically modifying the cross-linked high amylose starch, followed by
(c) gelatinization, and
(d) drying to obtain a powder of said controlled release excipient;
wherein said cross-linked high amylose starch is characterized in that less than about 20% of the amylose present in said high amylose starch prior to (a) is chemically cross-linked to amylopectin:

Another process for obtaining a cross-linked high amylose starch for this invention includes:
(a) cross-linking high amylose starch, followed by
(b) chemically modifying the cross-linked high amylose starch, followed by
(c) gelatinization, and
(d) drying to obtain a powder of said controlled release excipient;
wherein said cross-linked high amylose starch is characterized in that upon solubilization in 90% DMSO at 80°C. for about three days and gel permeation chromatography, less than about 20% of the amylose present prior to (a) is chemically cross-linked to and eluted with amylopectin.

Another process for obtaining a cross-linked high amylose starch for this invention includes:
(a) cross-linking high amylose starch, followed by
(b) chemically modifying the cross-linked high amylose starch, followed by
(c) gelatinization, and
(d) drying to obtain a powder of said controlled release excipient;
wherein said cross-linked high amylose starch is characterized in that upon solubilization in 90% DMSO at 80°C. for about three days and gel permeation chromatography, the height of the peak corresponding to amylose is higher than that of the peak corresponding to amylopectin-containing entities.

Another process for obtaining a cross-linked high amylose starch for this invention includes:
(a) cross-linking high amylose starch, followed by
(b) chemically modifying the cross-linked high amylose starch, followed by
(c) gelatinization, and
(d) drying to obtain a powder of said controlled release excipient;
wherein said cross-linked high amylose starch is characterized in that less than about 20% of the amylose present in said high amylose starch prior to (a) is chemically cross-linked to amylopectin.

Another process for obtaining a cross-linked high amylose starch for this invention includes:
(a) cross-linking high amylose starch, followed by
(b) chemically modifying the cross-linked high amylose starch, followed by
(c) gelatinization, and
(d) drying to obtain a powder of said controlled release excipient;
wherein said cross-linked high amylose starch is characterized in that upon solubilization in 90% DMSO at 80°C. for about three days and gel permeation chromatography, less than about 20% of the amylose present prior to (a) is chemically cross-linked to and eluted with amylopectin.

Another process for obtaining a cross-linked high amylose starch for this invention includes:
(a) cross-linking high amylose starch, followed by
(b) chemically modifying the cross-linked high amylose starch, followed by
(c) gelatinization, and
(d) drying to obtain a powder of said controlled release excipient;
wherein said cross-linked high amylose starch is characterized in that upon solubilization in 90% DMSO at 80°C. for about three days and gel permeation chromatography, the height of the peak corresponding to amylose is higher than that of the peak corresponding to amylopectin-containing entities.

Of course, a product having the structure of a cross-linked high amylose starch obtained by one of these processes, even though the manufacturing process is not identical to one of these is also within the scope of this invention.

The core of a formulation of this invention often includes a lubricant which is optionally hydrogenated vegetable oil.

In a preferred aspect, a formulation of the invention is a tablet formulated for oral administration.

In one particular embodiment, the invention is a solid dosage formulation that includes:
a core having a pharmacological agent dispersed in a first controlled-release matrix from which release of the agent is relatively slow; and
a coat formed over the core and comprising said agent dispersed in a second controlled-release matrix, the second controlled-release matrix comprising a physical mixture of polyvinyl acetate and polyvinylpyrrolidone and from which release of the agent is relatively fast.
In another embodiment, the invention provides a solid dosage formulation that includes:
a core comprising a pharmacological agent dispersed in a first controlled-release matrix comprising cross-linked high amylose starch, from which matrix release of the agent is relatively slow; and
a coat formed over the core and comprising a pharmacological agent in a second controlled-release matrix, the second controlled-release matrix comprising a physical mixture of polyvinyl acetate and polyvinylpyrrolidone, and wherein:
   release of the agent from the matrix of the core is relatively slow with respect to release of the agent from the matrix of the coat.

Another aspect of the invention is a solid dosage formulation that includes:
a core comprising a pharmacological agent in a first controlled-release matrix; and
a coat formed over the core and comprising a pharmacological agent in a second controlled-release matrix, the second controlled-release matrix comprising a physical mixture of polyvinyl acetate and polyvinylpyrrolidone, and wherein:
   release of the agent from the matrix of the core is relatively slow with respect to release of the agent from the matrix of the coat.

In another aspect, the invention includes a solid dosage formulation comprising a pharmacological agent for release thereof over an extended period of time, the formulation comprising:
a core comprising agent in a first controlled-release release matrix, the controlled-release matrix comprising cross-linked high amylose starch; and
a coat formed over the core and comprising the agent in a second controlled-release matrix, the second controlled-release matrix comprising a physical mixture of polyvinyl acetate and polyvinylpyrrolidone, and wherein:
   the agent is present in the core sufficient to obtain release into an aqueous environment, e.g. gastric juices, of no more than 50% of the agent from the formulation within one quarter of the period.

In such a formulation, the period can be between about 12 and about 24 hours, and between about 30% and about 70% of the agent is in the core. The agent in the first matrix and the agent in the second matrix is preferably soluble in water at least to the extent of 1 g/L, or more than 10 g/L, or more than 100 g/L, or more than 500 g/L, or more than 1000 g/L, or more than 2000 g/L. The agent can be an analgesic.

A particular embodiment of the invention includes a solid dosage formulation for use for a period of every four hours, or every six hours, every eight hours, every twelve hours, or every twenty-four hours, the formulation comprising:
a compressed core comprising a pharmacological agent including an amino group, the agent being present as a pharmacologically acceptable salt and being dispersed in a first controlled-release matrix comprising cross-linked high amylose starch; and
a coat formed by compression over the core and comprising the agent in a second coritrolled-release matrix, the second controlled-release matrix comprising a physical mixture of polyvinyl acetate and polyvinylpyrrolidone, and wherein:
   release of the agent from the formulation over the period includes a first phase with the average rate of release over the first 5% of the period being between three and eight times the rate of release of the agent half way through the period.

In a particular aspect of this particular embodiment, the ratio of the agent in core to the agent in the coat (w/w) is between 0.2 and about 7, the core is between 20% and 80% by-weight agent, the coat is between 15% and 50% by weight agent, and the ratio of the matrix of the coat to the agent of the coat (w/w) is between 0.3 and 7. Further, the preferred agent is tramadol, and preferably the coat includes a binding agent.

In another aspect, the invention is a controlled released tablet comprising:
a compressed core comprising cross-linked high amylose starch having tramadol, or a salt thereof, embedded therein; and
a coat formed over the core by compression, and comprising a physical mixture of polyvinyl acetate, polyvinylpyrrolidone, a binder, tramadol; and wherein:
   the ratio of the core/coat (w/w) is between about 0.2 and 0.6;
   the ratio of the tramadol in the core to the tramadol in the coat is between about 0.7 and about 1;
   the ratio of polyvinyl acetate/polyvinylpyrrolidne (w/w) is between about 6:4 and 9:1; and
   the rate of release of tramadol from the coat matrix is at least twice the rate of release of tramadol from the core when measured by a USP Type I apparatus in 50 mM phosphate, pH 6.8, and between 50 and 150 rpm.

The invention includes a method of manufacturing a controlled-release medication, the method comprising:
(i) blending a pharmacological agent and a first matrix material comprising a cross-linked high amylose starch;
(ii) forming the resultant blend of step (i) into a core;
(iii) blending a pharmacological agent and a second matrix material comprising a relatively fast release material with respect to the first matrix material;
(iv) forming the resultant blend of step (iii) as a coat onto the exterior of the core.

A method of manufacturing a controlled-release medication of invention can include:
(i) blending a pharmacological agent and a first matrix material;
(ii) forming the resultant blend of step (i) into a core;
(iii) blending a pharmacological agent and a second matrix material, the second matrix material comprising a physical mixture of polyvinyl acetate and polyvinylpyrrolidone and being a relatively fast release material with respect to the first matrix material;
(iv) forming the resultant blend of step (iii) as a coat onto the exterior of the core.

Step (ii) preferably comprises compressing the resultant blend of step
(i). Step (iii) can comprise compressing the resultant blend of step (iii) onto the exterior of the core. The agent in the core and the coat is preferably tramadol, the total amount of tramadol in the medication is effective as a daily dosage, and the medication comprises a formulation, as appropriate as defined within this specification.

The invention includes an oral tramadol pharmaceutical composition suitable for once daily administration comprising an effective amount of tramadol or a pharmaceutically acceptable salt thereof providing after a single administration *in vivo,* a median time to tramadol peak plasma concentration (Tₘₐₓ) between 2 and 8 hours and a mean peak tramadol plasma concentrations (Cₘₐₓ) which are less than three times the mean plasma concentration obtained 24 hours after administration (C₂₄ₕ) of a single dose of such composition.

Such a composition can be such that said mean peak tramadol plasma concentrations (Cₘₐₓ) are less than two times the mean plasma concentration obtained 24 hours after administration (C₂₄ₕ) of a single dose of such composition.

In another embodiment, the invention is an oral tramadol pharmaceutical composition suitable for successive administration, once daily, comprising an effective amount of tramadol or a pharmaceutically acceptable salt thereof providing *in vivo* a steady state in which, during a given 24 hour period, a tramadol maximum mean plasma concentration (Cₘₐₓ) of between 2 and 3 times a tramadol minimum mean plasma concentration (Cₘᵢₙ) is obtained. According to a particular aspect, the mean Cₘₐₓ is no greater than 350 ng/ml. Tthe mean plasma concentration of tramadol is preferably less than 90 percent of Cₘₐₓ for at least 18 hours of a said 24 hour period.

The invention includes a solid dosage formulation comprising:
a core comprising a pharmacological agent dispersed in a first controlled-release matrix comprising cross-linked high amylose starch; and
a coat formed over the core and comprising said agent dispersed in a second controlled-release matrix, different from the first such that release of the agent from the formulation is biphasic.

According to another aspect, the invention is a solid dosage formulation comprising:
a core comprising a pharmacological agent dispersed in a first controlled-release matrix; and
a coat formed over the core and comprising said agent dispersed in a second controlled-release matrix comprising a physical, mixture of polyvinyl acetate and polyvinylpyrrolidone such that release of the agent from the formulation is biphasic.

According to yet another aspect, the invention is a solid dosage formulation comprising:
a core comprising a pharmacological agent dispersed in a controlled-release matrix comprising a cross-linked high amylose starch; and
a coat formed over the core and comprising a pharmacological agent in a second controlled-release matrix comprising a physical mixture of polyvinyl acetate and polyvinylpyrrolidone.

In another embodiment, the invention is a solid dosage formulation comprising:
a core comprising about 50 mg, or about 75 mg or about 100 mg or about 125 mg or about 150 mg or about 175 mg or about 200 mg or about 225 mg or about 250 mg or about 275 mg or about 300 mg or about 325 mg or about 350 mg or about 375 mg or about 400 mg tramadol dispersed in a controlled-release matrix comprising a cross-linked high amylose starch; and
a coat formed over the core and comprising a pharmacological agent in a second controlled-release matrix comprising a physical mixture of polyvinyl acetate and polyvinylpyrrolidone.

The term "comprising" as used herein is used in its open-ended sense, unless the context would dictate otherwise. That is, a formulation comprising first and second matrices and an agent, for example, could thus also include other ingredients, such as a lubricant.

Formulations of the above-described formulations provide advantageous characteristics *in vivo,* as set out further below. Another aspect of the invention is thus a once daily oral pharmaceutical composition for controlled release of tramadol or a salt thereof, in which the composition, upon initial administration of one dose, provides an onset of analgesic effect within 2 hours, which analgesic effect continues for at least 24 hours after administration.

Another aspect of the invention is a once daily oral pharmaceutical composition for controlled release of tramadol or a salt thereof, wherein the composition, upon initial administration of one dose, provides a mean plasma concentration of at least 100 ng/mL within 2 hours of administration and continues to provide a mean plasma concentration of at least 100 ng/mL for at least 22 hours after administration.

Another aspect of the invention is a once daily oral pharmaceutical composition for controlled release of tramadol or a salt thereof, wherein the composition, upon initial administration of one dose, provides a mean plasma concentration of at least 100 ng/mL within 2 hours of administration and continues to provide a mean plasma concentration of at least 100 ng/mL for at least 23 hours after administration.

In another aspect, the invention is a once daily oral pharmaceutical composition for controlled release of tramadol or a salt thereof, wherein the composition, upon initial administration of one dose, provides a mean plasma concentration of at least 100 ng/mL within 2 hours of administration and continues to provide a mean plasma concentration of at least 100 ng/mL for at least 24 hours after administration.

A once daily oral pharmaceutical composition of the invention, in a preferred aspect, includes about 200 mg of tramadol or a salt thereof.

In yet another aspect, the invention is a once daily oral pharmaceutical composition for controlled release of tramadol or a salt thereof comprising 100 mg of tramadol or a salt thereof, wherein the composition, upon initial administration of one dose, provides a mean plasma concentration of at least 50 ng/mL within 2 hours of administration and continues to provide a mean plasma concentration of at least 50 ng/mL for at least 22 hours after administration.

: According to another aspect, the invention provides a once daily oral pharmaceutical composition for controlled release of tramadol or a salt thereof comprising 100 mg of tramadol or a salt thereof, wherein the composition, upon initial administration of one dose, provides a mean plasma concentration of at least 50 ng/mL within 2 hours of administration and continues to provide a mean plasma concentration of at least 50 ng/mL for at least 23 hours after administration.

Another aspect of the invention is a once daily oral pharmaceutical composition for controlled release of tramadol or a salt thereof comprising 300 mg of tramadol or a salt thereof, wherein the composition, upon initial administration of one dose, provides a mean plasma concentration of at least 150 ng/mL within 2 hours of administration and continues to provide a mean plasma concentration of at least 150 ng/mL for at least 22 hours after administration.

Another aspect of the invention is a once daily oral pharmaceutical composition for controlled release of tramadol or a salt thereof comprising 300 mg of tramadol or a salt thereof, wherein the composition, upon initial administration of one dose, provides a mean plasma concentration of at least 150 ng/mL within 2 hours of administration and continues to provide a mean plasma concentration of at least 150 ng/mL for at least 23 hours after administration.

Another aspect of the invention is a once daily oral pharmaceutical composition for controlled release of tramadol or a salt thereof comprising 300 mg of tramadol or a salt thereof, wherein the composition, upon initial administration of one dose, provides a mean plasma concentration of at least 150 ng/mL within 2 hours of administration and continues to provide a mean plasma concentration of at least 150 ng/mL for at least 24 hours after administration.

In another aspect of the invention is a once daily oral pharmaceutical composition for controlled release of tramadol or a salt thereof comprising 200 mg of tramadol or a salt thereof, wherein upon initial administration of 400 mg, the composition provides a mean plasma concentration of at least 200 ng/mL for at least 22 hours after administration.

Another aspect of the invention is a once daily oral pharmaceutical composition for controlled release of tramadol or a salt thereof comprising 200 mg of tramadol or a salt thereof, wherein upon initial administration of 400 mg, the composition provides a mean plasma concentration of at least 190 ng/mL for at least 23 hours after administration.

Another aspect of the invention is a once daily oral pharmaceutical composition for controlled release of tramadol or a salt thereof comprising 200 mg of tramadol or a salt thereof, wherein upon initial administration of 400 mg, the composition provides a mean plasma concentration of at least 180 ng/mL for at least 24 hours after administration.

The invention also provides a once daily oral pharmaceutical composition wherein the mean maximum plasma concentration (Cₘₐₓ) is less than 100 ng/mL

Further, a once daily oral pharmaceutical composition of the invention can provide a mean maximum plasma concentration (Cₘₐₓ) less than 300 ng/ml, or a mean maximum plasma concentration (Cₘₐₓ) less than 200 ng/mL.

A once daily oral pharmaceutical composition of the invention can be such that the mean maximum plasma concentration (Cₘₐₓ) is less than 2.2 times the mean plasma concentration obtained 24 hours after administration (C₂₄ₕ).

The once daily oral pharmaceutical composition can be such that the mean maximum plasma concentration (Cₘₐₓ) is less than 300 ng/mL.

The mean maximum plasma concentration (Cₘₐₓ) can be less than two times the mean plasma concentration obtained 24 hours after administration (C₂₄ₕ).

The mean maximum plasma concentration (Cₘₐₓ) can be less than 2.3 times the mean plasma concentration obtained 24 hours after administration (C₂₄ₕ).

The once daily oral pharmaceutical composition of the invention can provide a median time to the mean maximum plasma concentration (tₘₐₓ) of between 2 and 10 hours, or between 3 and 6 hours, or between 5 and 6 hours.

The invention also provides a once daily oral pharmaceutical composition for controlled release of tramadol or a salt thereof comprising 200 mg of tramadol or a salt thereof, wherein the composition, upon initial administration of one dose, provides an O-desmethyltramadol mean plasma concentration of at least 24 ng/mL within 2 hours of administration and continues to provide an O-desmethyltramadol mean plasma concentration of at least 25 ng/mL for at least 24 hours after administration.

According to another embodiment, the invention provides a once daily oral pharmaceutical composition for controlled release of tramadol or a salt thereof comprising 100 mg of tramadol or a salt thereof, wherein the composition, upon initial administration of one dose, provides an O-desmethyltramadol mean plasma concentration of at least 11 ng/mL within 2 hours of administration and continues to provide an O-desmethyltramadol mean plasma concentration of at least 12 ng/mL for at least 24 hours after administration.

According to another embodiment, the invention provides a once daily oral pharmaceutical composition for controlled release of tramadol or a salt thereof comprising 300 mg of tramadol or a salt thereof, wherein the composition, upon initial administration of one dose, provides an O-desmethyltramadol mean plasma concentration of at least 32 ng/mL within 2 hours of administration and continues to provide an O-desmethyltramadol mean plasma concentration of at least 32 ng/mL for at least 24 hours after administration.

In another embodiment, the invention is a once daily oral pharmaceutical composition for controlled release of tramadol or a salt thereof comprising 200 mg of tramadol or a salt thereof, wherein upon initial administration of 400 mg, the composition provides an O-desmethyltramadol mean plasma concentration of at least 50 ng/mL within 2 hours of administration and continues to provide an O-desmethyltramadol mean plasma concentration of at least 50 ng/mL for at least 24 hours after administration.

One object of the present invention is to provide flexible dosing options for patients with different analgesic requirements with a once daily formulation.

One embodiment of the present invention is to provide a once daily formulation which upon initial ingestion of a dose of 100 mg would provide the desired early onset of action but achieve mean tramadol plasma concentrations of at least 45 ng/mL between 2 and 24 hours.

A further embodiment of the present invention is to provide a once daily formulation which upon initial ingestion of a dose of 200 mg would provide the desired early onset of action but achieve mean tramadol plasma concentrations of at least 100 ng/mL between 2 and 24 hours.

A further embodiment of the present invention is to provide a once daily formulation which upon initial ingestion of a dose of 300 mg would provide the desired early onset of action but achieve mean tramadol plasma concentrations of at least 150 ng/mL between 2 and 24 hours.

A further embodiment of the present invention is to provide a. once daily formulation which upon initial ingestion of a dose of 400 mg would provide the desired early onset of action but achieve mean tramadol plasma concentrations of at least 180 ng/mL between 2 and 24 hours.

A further embodiment of the present invention is to provide a once daily formulation which upon initial ingestion of a dose would provide a C'ₘₐₓ to dose ratio of from about 0.90 to about 1.0.

A further embodiment of the present invention is to provide a once daily formulation which upon initial ingestion of a dose would provide a tramadol plasma concentration which rises steadily until peak tramadol concentrations are attained at a Tₘₐₓ of about 4 hours to about 6 hours. Preferably, the Tₘₐₓ occurs at about 5 hours to about 5.5 hours.

A further embodiment of the present invention is to provide a once daily formulation which upon initial ingestion of a dose would provide a tramadol plasma concentration which, after Tₘₐₓ, declines in a slow but steady manner, reflecting continuing absorption in addition to elimination processes. Preferably, the decline in the tramadol plasma concentration after Tₘₐₓ occurs in a log-linear fashion with a mean apparent terminal elimination half-life of between about 5.5 hours and about 6.5 hours.

A further embodiment of the present invention is to provide a once daily formulation which upon initial ingestion of a dose would provide a tramadol plasma concentration which, after Tₘₐₓ, declines in a slow but steady manner, reflecting continuing absorption in addition to elimination processes, and which absorption continues for at least 20 hours after Tₘₐₓ.

A further embodiment of the present invention is to provide a once daily formulation which upon initial ingestion of a dose provides a tramadol plasma concentration which, after Tₘₐₓ, declines in a log-linear fashion with an apparent terminal elimination rate constant (λ_{z}) of about 0.12 h⁻¹ for the tramadol plasma concentration.

A further embodiment of the present invention is to provide a once daily formulation which upon initial ingestion of a dose would provide a mean residence time (MRT) of tramadol ranging from about 15 hours and about 18 hours.

A further embodiment of the present invention is to provide a once daily formulation which upon initial ingestion of a dose would provide a half value duration (HVD) of tramadol which ranges from about 22.5 hours to about 25.4 hours.

A further embodiment of the present invention is to provide a once daily formulation which upon initial ingestion of a dose would provide a C'ₘₐₓ to AUC_{0-∞} ratio of from about 0.04 h⁻¹ to about 0.06 h⁻¹. Preferably, the C'ₘₐₓ to AUC_{0-∞} ratio is from about 0.04 h⁻¹ to about 0.05 h⁻¹. The ratio C'ₘₐₓ/AUC_{0-∞} is used for evaluating the rate of drug absorption.

A further embodiment of the present invention is to provide a once daily formulation which upon initial ingestion of a dose would provide a mean AUC₀₋₂₄ with respect to the tramadol plasma concentration which increases proportionally with dose over the range of dosage strengths of 100 mg to 300 mg of the controlled release composition.

A further embodiment of the present invention is to provide a once daily formulation which upon initial ingestion of a dose of 100 mg would provide a mean AUC_{0-Tmax} of from about 610 ng·h/mL to about 630 ng·h/mL.

A further embodiment of the present invention is to provide a once daily formulation which upon initial ingestion of a dose of 200 mg would provide a mean AUC_{0-Tmax} of from about 910 ng·h/mL to about 920 ng·h/mL.

A further embodiment of the present invention is to provide a once daily formulation which upon initial ingestion of a dose of 300 mg would provide a mean AUC_{0-Tmax} of from about 1570 ng·h/mL to about 1590 ng·h/mL.

A further embodiment of the present invention is to provide a once daily formulation which upon initial ingestion of a dose provides a mean ratio of AUC_{0.24}/AUC_{0-∞} of tramadol plasma concentration which ranges between about 70% and about 85%. Preferably, the mean ratio of AUC₀₋₂₄/AUC_{0-∞} of tramadol plasma concentration ranges between about 74% and about 80%. As a result, about 15% to about 30% of the administered dose is still circulating in the plasma 24 hours post-dose, depending on the dose administered.

A further embodiment of the present invention is to provide a once daily formulation which upon initial ingestion of a dose would provide a ratio of the C'ₘₐₓ to the dose released to the blood plasma in the first 24 hours (that is, AUC₀₋₂₄/AUC_{0-∞} multiplied by the dose) of from about 1.10 to about 1.35. Preferably the ratio is from about 1.15 to about 1.31.

A further embodiment of the present invention is to provide a once daily formulation which upon initial ingestion of a dose, would provide a ratio of the C'ₘₐₓ /Tₘₐₓ to the dose administered of from about 0.10 to about 0.20. Preferably the ratio is from about 0.12 to about 0.19.

A further embodiment of the present invention is to provide a once daily formulation which upon initial ingestion of a dose would provide a slope in ng/mi-hr following the peak blood plasma concentration level, which does not exceed a factor of about 0.035 of the total dose administered in mg. Preferably, the factor is about 0.03.

A further embodiment of the present invention is to provide a once daily formulation which upon initial ingestion of a dose would provide a ratio of the C'ₘₐₓ calculated with respect to the blood plasma concentration of O-desmethyltramadol, to the dose of tramadol from about 0.19 to about 0.22. Preferably the ratio is from about 0.20 to 0.21.

A further embodiment of the present invention is to provide a once daily formulation which upon initial ingestion of a dose would provide an O-desmethyltramadol plasma concentration which rises steadily until peak tramadol concentrations are attained at a Tₘₐₓ of about 8 hours to about 16 hours.

A further embodiment of the present invention is to provide a once daily formulation which upon initial ingestion of a dose would provide an O-desmethyltramadol plasma concentration which, after Tₘₐₓ, declines in a slow but steady manner, reflecting continuing tramadol absorption and subsequent metabolite formation in addition to elimination processes. Preferably, the decline in the O-desmethyltramadol plasma concentration occurs in a log-linear fashion with a mean apparent terminal elimination half-life of between about 6.7 hours and about 8.1 hours.

A further embodiment of the present invention is to provide a once daily formulation which upon initial ingestion of a dose would provide, after Tₘₐₓ, the formation of metabolite for at least 18 hours.

A further embodiment of the present invention is to provide a once daily formulation which upon initial ingestion of a dose would, after Tₘₐₓ, provide a decline in the O-desmethyltramadol plasma concentration in a log-linear fashion with an apparent terminal elimination rate constant (λ_{z}) for O-desmethyltramadol concentration of about 0.1 h⁻¹.

A further object of the invention is to provide a once daily formulation which upon initial ingestion of 100 mg, 200 mg and 300 mg strengths provides a half value duration (HVD) of O-desmethyltramadol plasma concentration which ranges from 25.6 to 28.1 hours.

A further embodiment of the present invention is to provide a once daily formulation which upon initial ingestion of a dose would provide a half value duration (HVD) of O-desmethyltramadol which ranges from about 25.6 hours to about 28.1 hours.

A further embodiment of the present invention is to provide a once daily formulation which upon initial ingestion of a dose would provide a C'ₘₐₓ to AUC_{0-∞} ratio calculated with respect to the O-desmethyltramadol plasma concentration, of about 0.04 h⁻¹. The ratio C'ₘₐₓ/AUC_{0-∞} is used for evaluating the rate of metabolite formation.

A further embodiment of the present invention is to provide a once daily formulation which upon initial ingestion of a dose would provide a mean AUC₀₋₂₄ calculated with respect to the O-desmethyltramadol plasma concentration, which increases proportionally with dose over the range of dosage strengths of 100 mg to 300 mg of the controlled release composition.

A further embodiment of the present invention is to provide a once daily formulation which upon initial ingestion of a dose of 100 mg would provide a mean AUC_{0-Tmax} with respect to the O-desmethyltramadol plasma concentration of from about 175 ng·h/mL to about 180 ng·h/mL.

A further embodiment of the present invention is to provide a once daily formulation which upon initial ingestion of a dose of 200 mg would provide a mean AUC_{0-Tmax} with respect to the O-desmethyltramadol plasma concentration of from about 530 ng·h/mL to about 550 ng·h/mL.

A further embodiment of the present invention is to provide a once daily formulation which upon initial ingestion of a dose of 300 mg would provide a mean AUC_{0-Tmax} with respect to the O-desmethyltramadol plasma concentration of from about 580 ng-h/mL to about 590 ng·h/mL.

A further embodiment of the present invention is to provide a once daily formulation which upon initial ingestion of a dose provides a mean ratio of AUC₀₋₂₄/AUC_{0-∞} of O-desmethyltramadol plasma concentration which ranges between about 65% and about 80%. Preferably, the mean ratio of AUC₀₋₂₄/Auc_{0-∞} of O-desmethyltramadol plasma concentration ranges between about 68% and about 75%. As a result, about 25% to about 32% of the active metabolite is still circulating in the plasma 24 hours post-dose.

A further embodiment of the present invention is to provide a once daily formulation which upon initial ingestion of a dose would provide a ratio of the C'ₘₐₓ calculated with respect to the O-desmethyltramadol plasma concentration, to the O-desmethyltramadol blood plasma concentration in the first 24 hours (AUC₀₋₂₄/AUC_{0-∞} multiplied by the dose of tramadol) of from about 0.0025 to about 0.0035. Preferably the ratio is from about 0.0027 to about 0.0031.

The present invention may be understood more fully by reference to the following detailed description and illustrative examples which are intended to exemplify non-limiting embodiments of the invention.

### DESCRIPTION OF THE DRAWINGS

Various features and advantages of the present invention will become clear from the more detailed description given below with reference to the accompanying drawings, in which:

**Figure 1****:** Flow diagram showing manufacturing process for tablets.

**Figure 2****:** Dissolution profiles (% released) of formulations A, B and C over 24 hours: *In vitro* performance of formulations A, B and C: under USP Type 1 Conditions; sodium phosphate buffer, 50 mM, pH 6.8, 100 rpm. 6 tablets were tested per time point.

**Figure 3(a)**: Mean tramadol plasma concentrations (ng/ml) for 48 hours following administration of 2 x 200 mg doses of composition (formulation B) (▲), and 1 x 200 mg Topalgic® LP BID q12h (Δ). Plasma concentrations were determined using an HPLC/UV assay.

**Figure 3(b)****:** Mean O-desmethyltramadol plasma concentrations (ng/ml) for 48 hours following a single administration of 1. x 200 mg dose of the composition (formulation B) (●), 2 x 200 mg dose of the composition (▲), 1 x 100 mg Topalgic® LP BID q12h (○), and 1 x 200 mg Topalgic® LP BID q12h (Δ).

**Figure 4(a)**: Plasma tramadol concentrations (ng/ml) of 27 subjects for 48 hours following a single administration of either 100 mg **(◆),** 200 mg (O), or 300 mg (Δ) strength tramadol formulations (A, B, and C, respectively).

**Figure 4(b)****:** Plasma ○-desmethyl tramadol concentrations (ng/ml) of 27 subjects for 48 hours following a single administration of either 100 mg (◆), 200 mg (○), and 300 mg (Δ) strength tramadol formulations (A, B, and C, respectively).

**Figure 5****:** Mean steady-state plasma tramadol (●) and O-desmethyl tramadol (○) concentrations (ng/ml) of 26 subjects dosed with the 200 mg tramadol, formulation B, and steady-state plasma tramadol (▲) and O-desmethyl tramadol (Δ) concentrations of 26 subjects dosed with Topalgic LP 100 mg BID.

### DETAILED DESCRIPTION OF THE INVENTION

### CORE

The core of a tablet of the invention includes at least one active ingredient and a matrix, these components associated in such a way that release of the pharmaceutical ingredient from the matrix is controlled. In a specific embodiment, the matrix of the core is a cross-linked high amylose starch known under the name Contramid^{®}, and described most recently in U.S. Patent No. 6,607,748 (Lenaerts et al.), which issued August 19, 2003. A preferred formulation in the context of this invention is provided in the specification of U.S. Patent No. 6,607,748.

Preferably, the core is formed by admixing the ingredients (in granular or powder form) and then compressing the mixture to form the core over which the coat is subsequently formed. The weight of the core can be any percentage of the weight of the total composition between 10% and 80%. The preferred percentage depends, upon other things, the total dosage of the pharmaceutical agent. In a particular embodiment described further below, a tablet contains 100 mg tramadol hydrochloride and the core is about 26% of the total weight of the tablet. In another embodiment, a tablet contains 200 mg tramadol hydrochloride and the core makes up about 33% of the total weight of the tablet. In yet another embodiment, a tablet contains 300 mg tramadol hydrochloride, and the core contributes 33% to the total weight of the tablet.

### Active Agent In the Core

An active pharmaceutical ingredient is present in the core of the composition of the present invention. A suitable pharmaceutical ingredient of the present invention is any such ingredient that is desired to be delivered in a sustained-release dosage form. A comprehensive list of suitable pharmaceutical agents can be found in The Merck Index. 12th Ed. Preferably, the pharmaceutical ingredient is, but not limited to, isonicotinic acid hydrazide, sodium salicylate, pseudoephedrine hydrochloride, pseudoephedrine sulfate, acetaminophen or diclofenac sodium, verapamil, glipizide, nifedipine, felodipine, betahistine, albuterol, acrivastine, omeprazole, misoprostol, tramadol, oxybutynin, trimebutine, ciprofloxacin, and salts thereof. In addition, the pharmaceutical agent can be an antifungal agent, such as ketoconazole, or an analgesic agent such as acetylsalicylic acid, acetaminophen, paracetamol, ibuprofen, ketoprofen, indomethacin, diflunisal, naproxen, ketorolac, diclofenac, tolmetin, sulindac, phenacetin, piroxicam, mefamanic acid, dextromethorphan, other non-steroidal anti-inflammatory drugs including salicylates, pharmaceutically acceptable salts thereof or mixtures thereof. Pro-drugs are part of the invention.

The solubility of the pharmaceutical agent in aqueous solution can be a wide variety of values. The aqueous solubility of the pharmaceutical agent can be less than 10⁻³ g/L, more than 10⁻³ g/L, more than 10⁻² g/L, more than 10^{-1.} g/L, more than 1 g/L, more than 10 g/L, more than 100 g/L, more than 500 g/L, more than 1000 g/L, or more than 2000 g/L. Preferably, the solubility is more than 100 g/L More preferably, the solubility is more than 500 g/L. Most preferably, the solubility is more than 1000 g/L.

The pharmaceutical agent can meet a variety of dosage requirement. For example, the dosage requirement of the pharmaceutical agent can be less than 1 mg/dosage unit, more than 1 mg/dosage unit, more than 10 mg/dosage unit, more than 100 mg/dosage unit, more than 200-mg/dosage unit, more than 300 mg/dosage unit, more than 400 mg/dosage unit, more than 500 mg/dosage unit, or more than 1000 mg/dosage unit. Preferably, the pharmaceutical agent is more than 50 mg/dosage unit. More preferably, the pharmaceutical agent is 100 mg/dosage unit, or more, e.g. 150 mg/dosage unit, or 200 mg/dosage unit, or 250 mg/dosage unit, or 300 mg/dosage unit, or more.

Particular embodiments include a core containing tramadol hydrochloride in which the core contains between about 10% and 90% of the total tramadol present in the tablet, e.g. about 45 mg of a 100 mg strength tablet (45% of the tablet total), or about 90 of a 200 mg strength tablet (45% of the tablet total), or about 151 mg of a 300 mg strength tablet (50% of the tablet total).

### Matrix of the Core

The release from the formulation of an active pharmaceutical ingredient located in the core is slower than the release of an active pharmaceutical ingredient located in the matrix of the coat. A preferred matrix of the core is cross-linked high amylose starch, known under the name Contramid® and described in U.S. Patent No. 6,607,748. In particular embodiments, the matrix makes up between about 10% and about 90% by weight of the core i.e., the ratio of the matrix of the core to the active ingredient of the core (w/w) is between about 0.1 and about 10, or between about 0.2 and about 9, or between about 0.2 and about 8, or between about 0.3 and about 7, or between about 0.4 and about 6, or between about 0.5 and about 5, or between about 0.6 and about 4, or between about 0.7 and about 4 or between about 1 and about 4, or between about 1 and about 3 and about 1.5 and about 2.5. In one particular embodiment, the core totals about 90 mg, of which about 44 mg is Contramid^{®}, and 45 mg is tramadol hydrochloride. In this case, Contramid^{®} thus makes up about 49 weight percent of the core.

### Optional Components

The core composition of the present invention may optionally include a pharmaceutically acceptable carrier or vehicle. Such carriers or vehicles are known to those skilled In the art and are found, for example, In Remingtons's Pharmaceutical Sciences 14th Ed. (1970). Examples of such carriers or vehicles include lactose, starch, dicalcium phosphate, calcium sulfate, kaolin, mannitol and powdered sugar. Additionally, when required, suitable binders, lubricants, and disintegrating agents can be included. If desired, dyes, as well as sweetening or flavoring agents can be included.

The core composition of the present invention may optionally Include accessory Ingredients including, but not limited to dispersing agents such as microcrystalline cellulose, starch, cross-linked starch, cross-linked poly(vinyl pyrrolidone), and sodium carboxymethyl cellulose; flavoring agents; coloring agents; binders; preservatives; surfactants and the like.

The core can, optionally, also include one or more suitable binders known to one of ordinary skilled in the art.

Suitable forms of microcrystalline cellulose, for example, MCC-PH101, MCC-102, MCC-105, etc.

Suitable lubricants, such as those known to the skilled person, may also be included. For example, magnesium stearate, vegetable oil, talc, sodium-stearyl fumarate, calcium stearate, stearic acid, etc.

Suitable glidants, known in the art, may also be included. Examples of such glidants include, but are not limited to talc, colloidal silicon dioxide, etc.

### Proportion

The active agent is present at levels ranging from about 1 to about 90 wt.% of the total weight of the core, preferably from about 10 to about 70 wt% of the total composition of the core, more preferably from about 20 to about 60 wt.% of the total composition of the core, and probably most often between about, 30 to about 50 wt.% of the total composition of the core.

Of course, the total amount of all components is 100 wt.%, and those of ordinary skill in the art can vary the amounts within the stated ranges to achieve useful compositions.

### COAT

The coat of the dosage form includes a physical mixture of polyvinyl acetate and polyvinylpyrrolidone and the active pharmaceutical ingredient(s) of the coat. The coat can also include a cross-linked high amylose: starch, e.g., Contramid^{®}, and other optional components. In a preferred embodiment, the coat is formed by dry compression. The weight of the coat can be any percentage of the weight of the total composition between about 10% and about 90%, but is preferably in the higher part of this range. The coat thus usually makes up between about 20% to about 90%, (w/w) of a tablet of the invention, or about 25% to about 90%, or about 30% to about 85%, or about 35 % to about 85%, or about 40% to about 85%, or about 45% to about 85%, or about 45% to about 90%, or about 50% to about 90% or about 50% to about 85 %, or about 55% to about 90%, or about 55% to about 85%, or about 55% to about 80%, or about 60% to about 90%, or about 60% to about 85%, or about 60% to about 80%, or about 60% to about 75%, or about 65% to about 90%, or about 65% to about 85%, or about 65% to about 80%, or about 65% to about 75%, or about 65% or about 70% or about 75%.
The coat often includes an optional binding agent.

### Polyvinyl Acetate and Polyvinylpyrrolidone of the Coat

The weight percentage of the polyvinyl acetate/polyvinylpyrrolidone mixture in the coat can be anywhere within a wide range of values. Depending on the solubility in water of the active ingredient in the coat, the amount of the polyvinyl acetate/polyvinylpyrrolidone mixture in the coat can be adjusted. United States Patent Publication No. 2001/0038852 describes ways In which such adjustments can be made. For example, for active ingredients that are soluble to extremely soluble in water, polyvinyl acetate/polyvinylpyrrolidone mixture can be about 20 to about 80 wt.% of the coat, preferably about 30 to about 65 wt.%, or about 40 to about 55wt.%. In a particular embodiment described below, Kollidon^{™} SR makes up about 45% by weight of a coat that is about 31 % by weight tramadol hydrochloride and about 23% xanthan gum. For active ingredients that are sparingly soluble to slightly soluble in water, the amount of polyvinyl acetate/polyvinylpyrrolidone mixture is often lower, as described in United States Patent Publication No. 2001/0038852.

The weight ratio of polyvinyl acetate to polyvinylpyrrolidone in the polyvinyl acetate/polyvinylpyrrolidone mixture can be a wide range of values. Preferably, such ratio is between about 6:4 and 9:1; more likely between about 7:3 and 6:1, even more preferably about 8:2.

The molecular weight of the polyvinyl acetate component in the polyvinyl acetate/polyvinylpyrrolidone mixture can be a wide range of values. Preferably, the average molecular weight of the polyvinyl acetate is about 100 to about 10,000,000; or about 1,000 to about 1,000,000; or about 10,000 to about 1,000,000; or about 100,000 to about 1,000,000; or about 450,000.

The molecular weight of the polyvinylpyrrolidone, component in the polyvinyl acetate/polyvinylpyrrolidone mixture can be a wide range of values. The average molecular weight of the polyvinylpyrrolidone can be from about 100 to about 10,000,000; or about 1,000 to about 1,000,000; or about 5,000 to about 500,000; or about 10,000 to about 100,000; or about 50,000.

The polyvinyl acetate and polyvinylpyrrolidone mixture can be prepared by a variety of processes including simply mixing powders of polyvinylpyrrolidone and polyvinyl acetate. In a preferred embodiment, such mixture is spray dried powder of a colloidal dispersion of polyvinyl acetate and polyvinylpyrrolidone solution. Optionally, sodium lauryl sulfate is used as a stabilizer in order to prevent agglomeration during spray drying process and/or colloidal silica is used to improve the flow properties of the polyvinyl acetate/polyvinylpyrrolidone mixture. Optionally, polyvinyl acetate and polyvinylpyrrolidone can be formed in a random or a block copolymer.

### Optional Components

Suitable binding agents for the present invention include, but are not limited to, plant extracts, gums, synthetic or natural polysaccharides, polypeptides, alginates, synthetic polymers, or a mixture thereof.

Suitable plant extracts to be used as gelling agents Include, but are not limited to, agar, ispaghula, psyllium, cydonia, ceratonia or a mixture thereof.

Suitable gums to be used as gelling agents include, but are not limited to, xanthan gum, guar gum, acacia gum, ghatti gum, karaya gum, tragacanth gum or a mixture thereof.

Suitable synthetics or natural hydrophilic polysaccharides to be used as gelling agents include, but are not limited to, hydroxyalkylcelluloses, cellulose ethers, cellulose esters, nitrocelluloses, dextrin, agar, carrageenan, pectin, furcellaran, starch or starch derivatives, cross-linked high amylose starch, or a mixture thereof.

Suitable polypeptides to be used as gelling agents include, but are not limited to, gelatin, collagen, polygeline or a mixture thereof.

Suitable alginates to be used as gelling agents include, but are not limited to, alginic acid, propylene glycol alginate, sodium alginate or a mixture thereof.

Suitable synthetic polymers to be used as gelling agents include, but are not limited to, carboxyvinyl polymer, polyvinyl alcohol, polyvinyl pyrrolidone, polyethelene oxide, polyethylene glycols, copolymers of ethylene oxide and propylene oxide and their copolymers or a mixture thereof.

In a preferred embodiment, the gelling agent is a gum such as xanthan gum, guar gum, acacia gum, ghatti gum, karaya gum, tragacanth gum or a mixture thereof, PEO 7,000,000 and HPMC K100 M.

In a most preferred embodiment, the gelling agent is xanthan gum.

### Active agent of the Coat

A suitable active pharmaceutical ingredient of the present invention is any active agent that it is desired to be delivered in a sustained-release dosage form. A comprehensive list of suitable pharmaceutical agents can be found in The Merck Index, 12th Ed. Preferably, the pharmaceutical agent is, but not limited to, isonicotinic acid hydrazide, sodium salicylate, pseudoephedrine hydrochloride, pseudoephedrine sulfate, acetaminophen or diclofenac sodium, verapamil, glipizide, nifedipine, felodipine, betahistine, albuterol, acrivastine, omeprazole, misoprostol, tramadol, oxybutynin, trimebutine, ciprofloxacin, and salts thereof. In addition, the pharmaceutical agent can be an antifungal agent, such as ketoconazole, or an analgesic agent such as acetylsalicylic acid, acetaminophen, paracetamol, ibuprofen, ketoprofen, indomethacin, diflunisal, naproxen, ketorolac, diclofenac, tolmetin, sulindac, phenacetin, piroxicam, mefamanic acid, dextromethorphan, other non-steroidal anti-inflammatory drugs including salicylates, pharmaceutically acceptable salts thereof or mixtures thereof.

The solubility of the pharmaceutical agent in aqueous solution can be a wide variety of values. The aqueous solubility of the pharmaceutical agent can be less than 10⁻³ g/L, more than 10⁻³ g/L, more than 10⁻² g/L, more than 10⁻¹ g/L, more than 1 g/L, more than 10 g/L, more than 100 g/L, more than 500 g/L, more than 1000 g/L, or more than 2000 g/L. Preferably, the solubility is more than 100 g/L. More preferably, the solubility is more than 500 g/L. or even 1000 g/L.

The pharmaceutical agent can meet a variety of dosage requirements. For example, the dosage requirement of the pharmaceutical agent can be less than 1 mg/dosage unit, more than 1 mg/dosage unit, more than 10 mg/dosage unit, more than 100 mg/dosage unit, more than 200 mg/dosage unit, more than 300 mg/dosage unit, more than 400 mg/dosage unit, more than 500 mg/dosage unit, or more than 1000 mg/dosage unit. Preferably, the pharmaceutical agent is more than 50 mg/dosage unit. More preferably, the pharmaceutical agent is more than 100 mg/dosage unit. Most preferably, the pharmaceutical agent is more than 200 mg/dosage unit.

The coat can be between about 5% and about 90% by weight active pharmaceutical ingredient, or between about 5% and about 80% by weight api, or between about 10% and about 70% by weight api, or between about 10% and about 60% by weight api, or between about 15% and about 50% by weight api, or between about 15% and about 45% by weight api, or between about 15% and about 40% by weight api, or between about 20% and about 35% by weight api, or between about 20% and about 30% by weight api.

In particular embodiments, described further below, the weight of tramadol from a 100 mg tramadol tablet is about 21% by weight of the coat. The weight of tramadol from a 200 mg tablet is about 31 % by weight of the coat. The weight of tramadol from a 300 mg tablet is about 30% by weight of the coat.

### ROUTES OF ADMINISTRATION

The tablet composition of the present invention can be administered through, but not limited to, a number of routes such as oral, sublingual, and rectal. The preferred route of administration of the compositions of the present invention is oral.

Compositions of the present invention that are suitable for oral administration may be presented as discrete units such as tablets or granules. Preferably, the compositions of the present invention are presented in a tablet form. Such tablets may be conventionally formed by compression or molding. Compressed tablets may be prepared by compressing in a suitable machine the mixture of one or more components described above. Molded tablets may be made by molding in a suitable machine the above components, which can be optionally moistened with an inert liquid diluent. The tablets may optionally be coated and/or have other identifying indicia visible to the consumer. A tablet can also be in a variety of forms, e.g., uncoated , dry coated, or film coated, etc. A tablet can also be in a variety of shapes (e.g., oval, sphere, etc.) and sizes. A comprehensive discussion of tablets can be found in references such as The Theory and Practice of Industrial Pharmacy by Lachman et al., 3rd Ed. (Lea & Febiger, 1986).

### Dissolution Profile of Sustained-Release Composition

The active agent of the composition exhibits the following *in vitro* dissolution profile when measured with a USP Type I apparatus in 50 mM phosphate, pH 6.8, and stirring between 50 and 150 rpm:

an average rate of between 10% and 30% per hour of the agent is released between 0 and 2 hours when tested *in vitro* using a USP Type I apparatus in 50 mM phosphate, pH 6.8, and stirring between 50 and 150 rpm; or

between 10% and 40% of the agent is released from the formulation between 0 and about 2 hours of measurement, between about 30% and 60% of the agent is released from the formulation between 2 and about 7 hours of the measurement, between about 50% and 80% of the agent is released from the formulation between 7 and about 12 hours of measurement, and between about 80% and 100% of the agent is released from the formulation after about 20 hours of measurement; or more preferably

between 15% and 35% of the agent is released from the formulation at 2 hours of measurement, between about 40% and 60% of the agent is released from the formulation between at 7 hours of the measurement, between about 60% and 80% of the agent is released from the formulation at 12 hours of measurement, and between about 85% and 100% of the agent is released from the formulation after about 20 hours of measurement, or

between 20% and 40% of the agent is released from the formulation at 2 hours of measurement, between about 40% and 60% of the agent is released from the formulation between at 7 hours of the measurement, between about 60% and 80% of the agent is released from the formulation at 12 hours of measurement, and between about 85% and 100% of the agent is released from the formulation after about 20 hours of measurement.

The present invention will be more readily understood by referring to the following examples which are given to illustrate the invention rather than to limit its scope.

### EXAMPLES

The cross-linked high amylose starch used in the these examples is made by a process comprising the steps of crosslinking and chemically modifying, followed by gelatinization and drying. Such process is described in more detail in U.S. Patent No. 6,607,748 (Lenaerts et al.*),* which issued August 19; 2003, and known in the marketplace under the name Contramid^{®}. and described in Examples I and II.

### Example I

### A. Cross-Linking

High amylose starch (30.0 kg) containing about 70% w/w of amylose (Cl AmyloGel 03003) is placed in a reactor. To this reactor is added water (55.01) containing sodium hydroxide (30.0 g) and sodium sulfate (2.40 kg). The resulting slurry is heated to a temperature of 30°C. Phosphorus oxychloride (22.5 g) is added to the reaction mixture which is reacted for one hour.

### B. Chemical Modification, Hydroxyproylation

The crude reaction mixture from Part A is transferred into a hydroxypropylation reactor. The reaction mixture is heated to 40°C. over 30 minutes and the reaction is purged with nitrogen. After a full purge, propylene oxide (1.80 kg) is added. The reaction mixture is kept at 40°C. for 20 hours. The reaction mixture is neutralized with 0.1N H₂SO₄ (1:2 v/v) to a pH of 5.5. The starch slurry is washed with a basket-centrifuge at a speed of 1200 rpm. The obtained starch cake is re-slurrified in 35 I of water and centrifuged a second time. The resulting starch cake is dried in a flash dryer at an inlet temperature of 160°C. and an outlet temperature of 60°C.

### C. Gelatinization

The modified granular starch cake is diluted in demineralized water in order to form a slurry at a concentration of about 8% calculated on dry substance. The resulting slurry has a relative density of 1.032 kg/l compared to water. The pH of the modified starch slurry is adjusted to 6.0. The slurry is then heated to 160°C. by direct steam injection (Schlick Model 825). The temperature variation is not higher than ±1°C. The slurry is held in a holding column for a period of 4 minutes at a temperature of 160°C. and a pressure of about 5.5 bar. The pressure is then reduced to atmospheric by passing through a flash. The slurry is then contained at 95°C. In a hold tank.

### D. Spray-Drying

The drying of the slurry from Part C is carried out using a Niro FSD 4 spray-drying tower equipped with a 0.8 mm nozzle and fed at 10 l/hour. The inlet temperature is fixed at 300°C. and the outlet temperature of 120°C. The obtained powder is a controlled release excipient with the following properties:

| Properties | |
|---|---|
| Moisture Content | 4.5% |
| Bulk Density | 150 g/l |
| Packed Density | 210 g/l |
| pH | 5.4 |
| Particle Size Peak Value | 50 *µ*m |
| (Laser Particle Sizer-Sympatec) | |

### Example II

### A. Cross-Linking

High amylose starch (30.0 kg) containing about 70% w/w of amylose (Cl AmyloGel 03003) is placed in a reactor. To this reactor is added water (55.01) containing sodium hydroxide (30.0 g) and sodium sulfate (2.40 kg). The resulting slurry is heated to a temperature of 30°C. Sodium trimetaphosphate (45 g) is added to the reaction mixture which is reacted for one hour.

### B. Chemical Modification, Hydroxyproylation

The crude reaction mixture from Part A is transferred into a hydroxypropylation reactor. The reaction mixture is heated to 40°C. over 30 minutes and the reaction is purged with nitrogen. After a full purge, propylene oxide (1.80 kg) is added. The reaction mixture is kept at 40°C. for 20 hours. The reaction mixture is neutralized with 0.1 N H₂SO₄ (1:2 v/v) to a pH of 5.5. The starch slurry is washed with a basket-centrifuge at a speed of 1200 rpm. The obtained starch cake is re-slurrified in 35 I of water and centrifuged a second time. The resulting starch cake is dried in a flash dryer at an inlet temperature of 160°C. and an outlet temperature of 60°C.

### C. Gelatinization

The modified granular starch cake is diluted in demineralized water in order to form a slurry at a concentration of about 8% calculated on dry substance. The resulting slurry has a relative density of 1.032 kg/l compared to water. The pH of the modified starch slurry is adjusted to 6.0. The slurry is then heated to 160°C. by direct steam injection (Schlick Model 825). The temperature variation is not higher than ±1°C. The slurry is held in a holding column for a period of 4 minutes at a temperature of 160°C. and a pressure of about 5.5 bar. The pressure is then reduced to atmospheric by passing through a flash. The slurry is then contained at 95°C. in a hold tank.

### D. Spray-Drying

The slurry from Part C is carried out using a Niro FSD 4 spray-drying tower equipped with a 0.8 mm nozzle and fed at 10 l/hour. The inlet temperature is fixed at 300°C. and the outlet temperature of 120°C. The obtained powder is a controlled release excipient with the following properties:

| Properties | |
|---|---|
| Moisture Content | 5.2% |
| Bulk Density | 103 g/l |
| Packed Density | 155 g/l |
| pH | 5.3 |
| Particle Size Peak Value | 70 µm |
| (Laser Particle Sizer-Sympatec) | |

Lubritab^{®} is a product sold by Penwest Pharmaceuticals Co. (Cedar Rapids, IA, USA). Kollidon^{™} SR is a product produced by BASF (Germany). Encompress^{™} is a dicalcium phosphate dihydrate which can be purchased from Mendell (Patterson, NY). Tramadol hydrochloride can be obtained from Chemagis Ltd., 3 Hashlosha Street, P.O. Box 9091, 61090, Tel Aviv, Israel. Methods of synthesis and purification of tramadol are described in; for example, U.S. Patent Nos., 3,652,589, 5,414,129, 5,672,755, 5,874,620, 5,877,351, and 6,169,205.

### Manufacturing Procedure

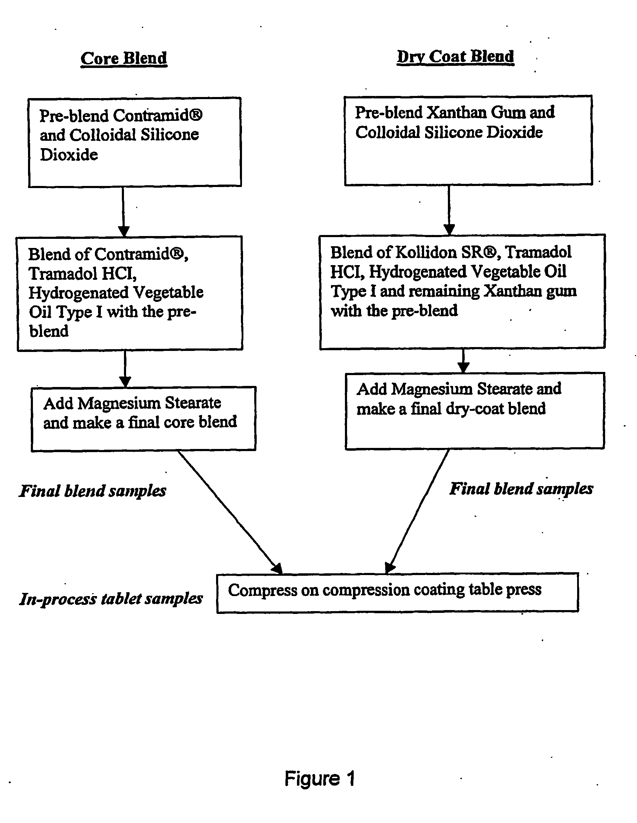

Tablets of the invention can be manufactured according to the process set out generally in the flow chart of Figure 1, and described in more detail below.

Weighing: Raw materials are dispensed into clearly labeled containers.

**Core Pre-Blend:** Blend a portion of the Contramid® and Colloidal Silicon Dioxide and pass through #30 mesh screen into a suitable container.

**Core Blend:** Place a portion of the Contramid® into a blender. Pass Tramadol Hydrochloride through a #30 mesh screen and' add to blender. Rinse container with a portion of Contramid® and add to blender. Sieve Hydrogenated Vegetable Oil Type I through a #30 mesh screen and add to the blender. Add the Core Pre-Blend into the blender. Add the remaining Contramid® into the blender, and blend all ingredients. Sieve the Magnesium Stearate through a #30 mesh screen and add blend with other ingredients. Dispense blend in suitable container and identify as Core Blend.

Dry Coated Pre-Blend: Blend a portion of the Xanthan Gum and all of the Colloidal Silicon Dioxide and pass through #30 mesh screen.

**Dry Coated Blend:** Place a portion of the Kollidon® SR into a blender. Pass Tramadol Hydrochloride through Kason Separator with a #30 mesh screen into suitable container and add to blender. Rinse container with remaining xanthan gum and add to blender. Sieve Hydrogenated Vegetable Oil Type 1 through a #30 mesh screen and add to the blender. Place Dry Coated Pre-Blend and the remainder of the Kollidon® SR. into the blender, and blend with all ingredients. Sieve the magnesium stearate through a #30 mesh screen and blend with other ingredients. Dispense granulation in suitable container and identify as Dry Coated Blend.

**Compression:** Use a Manesty Dry-Cota press to produce compression-coated tablets.

### Example 1

Formulations A, B, and C, as shown in Table 3, were manufactured according to the process set out above.

**Table 3: Recipes for Controlled Released Tramadol Formulations A, B and C.**

| **1) INGREDIENT** | **Formulation A** | | | **Formulation B** | | | **Formulation C** | |
|---|---|---|---|---|---|---|---|---|
| | | **%** | **mg/tablet** | | **%** | **mg/tablet** | **%** | **mg/tablet** |
| **Core** | | | | | | | | |
| Tramadol Hydrochloride | | 50 | 45 | | 50 | 90 | 63.25 | 151.8 |
| Contramid® | | 48.3 | 43.47 | | 48.3 | 86.94 | 35.05 | 84.1 |
| Hydrogenated Vegetable Oil | | 0.75 | 0.675 | | 0.75 | 1.35 | 0.75 | 1.8 |
| Silica | | 0.2 | 0.18 | | 0.2 | 0.36 | 0.20 | 0.5 |
| Magnesium Stearate | | 0.75 | 0.675 | | 0.75 | 1.35 | 0.75 | 1.8 |
| **Core Total Weight** | | 100 | 90 | | 100 | 180 | 100 | 240 |

| **2) COAT** | | | | | | | | |
|---|---|---|---|---|---|---|---|---|
| Tramadol Hydrochloride | | 21.15 | 55 | | 30.56 | 110 | 30.56 | 148.5 |
| Silica | | 0.20 | 0.52 | | 0.20 | 0.72 | 0.20 | 1.0 |
| Kollidon SR® | | 51.42 | 133.7 | | 45.16 | 162.58 | 45.16 | 219 |
| Xanthan Gum | | 25.72 | 66.86 | | 22.58 | 81.3 | 22.58 | 109.5 |
| Hydrogenated Vegetable Oil | | 1.00 | 2.6 | | 1.00 | 3.6 | 1.00 | 4.9 |
| Magnesium Stearate | | 0.50 | 1.3 | | 0.50 | 1.8 | 0.50 | 2.4 |
| **Coat Total Weight** | | 100 | 260 | | 100.00 | 360 | 100 | 485 |

| **3) COATED TABLET** | | | | | | | | |
|---|---|---|---|---|---|---|---|---|
| Tramadol Hydrochloride | | 28.57 | 100 | | 37.04 | 200 | 41.38 | 300 |
| Contramid® | | 12.42 | 43.47 | | 16.10 | 86.94 | 11.60 | 84.1 |
| Hydrogenated Vegetable Oil | | 0.94 | 3.275 | | 0.92 | 4.95 | 0.92 | 6.7 |
| Silica | | 0.20 | 0.7 | | 0.20 | 1.08 | 0.20 | 1.5 |
| Magnesium Stearate | | 0.56 | 1.975 | | 0.58 | 3.15 | 0.58 | 4.2 |
| Kollidon SR® | | 38.20 | 133.7 | | 30.11 | 162.58 | 30.21 | 219 |
| Xanthan Gum | | 19.11 | 66.86 | | 15.06 | 81.3 | 15.10 | 109.5 |
| Coated Tablet TotalWeight: | | 100 | 350 | | 100 | 540 | 100 | 725 |

Dissolution profiles of formulations A, B and C are shown in Figure 2.

### BIOAVAILABILITY

### SINGLE ADMINISTRATION

### Example 2

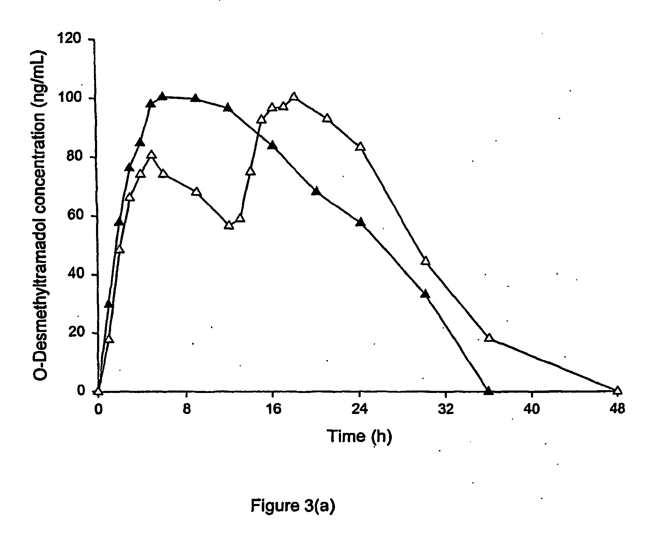

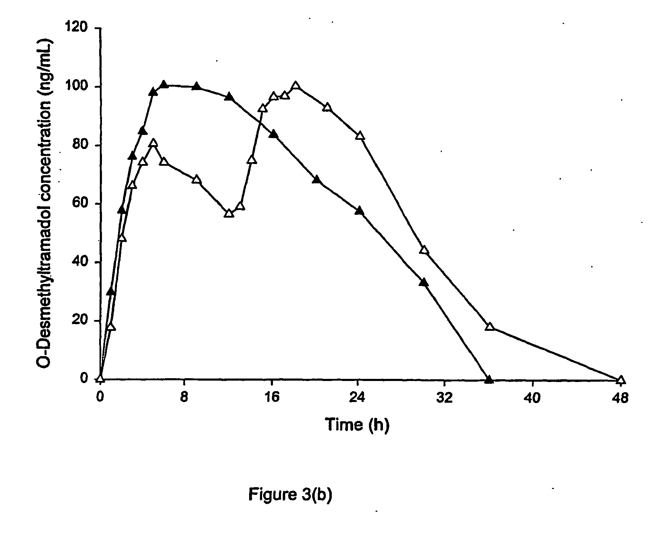

The plasma pharmacokinetic profile of tramadol and its principal metabolite, O-desmethyltramadol, after a single oral administration of 200 mg, (formulation B) was determined in comparison to a currently available 100 mg formulation, Topalgic® administered two times a day, and after a double dose administration of 200 mg, (formulation B) was determined in comparison to a currently available 200 mg formulation, Topalgic® administered two times a day. The study was an open, single dose, randomized, three-way cross-over design with at least a 7 day wash-out period between each administration. Results are shown in Figures 3(a) and 3(b).

### Example 3

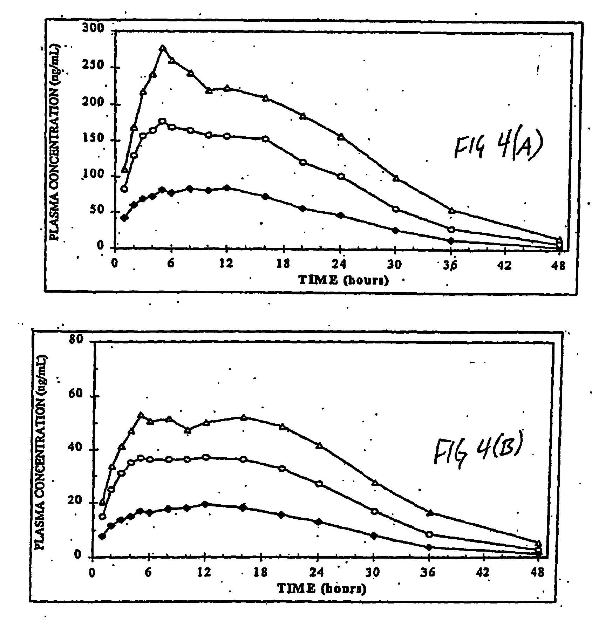

The plasma pharmacokinetic profile of tramadol and its principal metabolite, O-desmethyltramadol, after a single oral administration of 100, 200 and 300 mg, formulations A, B and C, respectively, was determined. The study was an open, single dose, randomized, three-way cross-over design with at least a 7 day wash-out period between each administration. Results are shown in Figures 4(a) and 4(b).

A median time to tramadol peak plasma concentration (Tₘₐₓ) of between 2 and 8 hours and a mean peak tramadol plasma concentration (Cₘₐₓ) which is less than three times the mean plasma concentration obtained 24 hours after administration (C₂₄ₕ) of a single dose of the composition was obtained. In a narrower sense, the peak tramadol plasma concentration (Cₘₐₓ) obtained in each case is less than two times the plasma concentration obtained 24 hours after administration (C₂₄ₕ) of a single dose of a composition of the invention.

### Example 4

### Steady-State

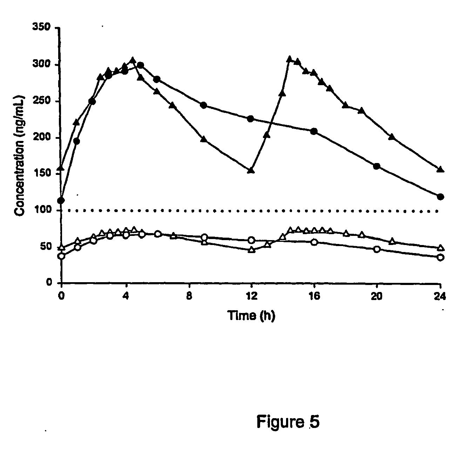

The steady state plasma pharmacokinetic profile of tramadol and its principal metabolite, O-desmethyltramadol, following daily administration of 200 mg, formulation B, was determined. The profile was obtained in an open-label, two-period crossover randomized study. Results obtained are shown in Figure 5.

The invention provides an oral tramadol pharmaceutical composition suitable for successive administration, once daily, comprising an effective amount of tramadol *in vivo* in a steady state in which, during a given 24 hour period, a tramadol maximum plasma concentration (Cₘₐₓ) of between 2 and 3 times a tramadol minimum plasma concentration (Cₘᵢₙ) is obtained. More particularly, an average Cₘₐₓ of no greater than 350 ng/ml is achievable. Further, a plasma concentration of tramadol of less than 90 percent of Cₘₐₓ for at least 18 hours of the 24 hour period can be achieved, on average.

The term "λ_{z}" is the apparent terminal elimination rate constant, determined by the slope of the regression during the log-linear phase.

The term "AUC_{0-Tmax}" is the mean area under the plasma concentration-time curve from time 0 to Tₘₐₓ and is used as an indicator of the rate of drug absorption, or metabolite formation. It is calculated as the arithmetic mean of the area under the plasma concentration-time curve from time 0 to Tₘₐₓ calculated for each individual participating in the bioavailability study.

The term "AUC_{0-∞}" is the mean area under the plasma concentration-time curve extrapolated to infinity. It is calculated as the arithmetic mean of the area under the plasma concentration-time curve from time 0 extrapolated to infinity, for each individual participating in the bioavailability study.

The term "C'ₘₐₓ" is the maximum observed plasma concentration, calculated as the mean of the individual maximum blood plasma concentrations.

The term "half-life" is the apparent terminal elimination half-life.

The term "HVD" is the half value duration, that is, the time during which tramadol concentrations are above one half the C'ₘₐₓ. This parameter is an indicator of the shape of the plasma concentration time curve, that is, the larger the value of HVD, the better the controlled release.

The term "MRT" is the mean residence time, which is an estimate of the average time that a tramadol molecule resides in the body following oral administration.

The term "tₘₐₓ" is the time at which Cₘₐₓ is achieved.

The term "Tₘₐₓ " is the time at which the maximum blood plasma concentration is observed for each individual participating in the bioavailability study.

The term "Rstart" is the time at which plasma concentrations begin to decline in a log-linear fashion, that is, the time at which either drug absorption or metabolite formation is complete.

Tramadol pharmacokinetic parameters of the controlled release composition are presented in Table 4, and O-desmethyltramadol pharmacokinetic parameters of the controlled release composition are presented in Table 5.

**Table 4. Summary of Tramadol Pharmacokinetic Parameters**

| Formulation Strength (mg) | Dose (mg) | Descriptive Statistic | C'ₘₐₓ (ng/mL | AUC_{0-∞} (ng:h/mL) | AUC_{0-Tmax} (ng·h/mL) | C'ₘₐₓ/AUC_{0-∞} (h⁻¹) | λ_{z} (h⁻¹) | Rstart (h) | half-life (h) | MRT (h) | HVD (h) | AUC₀₋₂₄ (ng·h/mL) | AUC_{0- 24}/AUC_{0-∞} (%) |
|---|---|---|---|---|---|---|---|---|---|---|---|---|---|
| 100 | 100 | Arith. mean | 91.03 | 2108 | 625 | 0.0442 | 0.118 | 21.2 | 6.11 | 16.03 | 22.5 | 1635 | 78.9 |
| | | SD | 26.83 | 731 | 471 | 0.0052 | 0.02 4 | 4.3 | 1.31 | 2.13 | 3.4 | 465 | 6.60 |
| 200 | 200 | Arith. mean | 196.55 | 4416 | 915 | 0.0455 | 0.11 8 | 22.9 | 6.11 | 16.4 6 | 23.5 | 3374 | 77.2 |
| | | SD | 58.33 | 1192 | 567 | 0.0108 | 0.02 5 | 5.0 | 1.26 | 2.28 | 4.5 | 860 | 8.1 |
| 300 | 300 | Arith. mean | 290.08 | 6741 | 1578 | 0.0432 | 0.11 5 | 24.8 | 6.30 | 17.6 0 | 25.4 | 4900 | 73.9 |
| | | SD | 147.16 | 2156 | 1338 | 0.0126 | 0.02 3 | 4.4 | 1.52 | 3.03 | 6.6 | 1544 | 10.1 |
| 200 | 400 | Arith. mean | 487.35 | 9332 | NC | 0.0544 | 0.12 0 | 21.1 | 6.11 | 15.3 3 | NC | 7471 | 80.0 |
| | | SD | 210.43 | 3767 | NC | 0.0198 | 0.02 7 | 6.5 | 1.53 | 2.83 | NC | 2887 | 10.1 |

| | | | | | | | | | | | | | |
|---|---|---|---|---|---|---|---|---|---|---|---|---|---|
| NC- Not calculated | | | | | | | | | | | | | |

**Table 5. Summary of O-desmethyltramadol Pharmacokinetic Parameters**

| Formulation Strength (mg) | Dose (mg) | Descriptive Statistic | C'ₘₐₓ (ng/mL | AUC_{0-∞} (ng:h/mL) L) | AUC_{0-Tmax} (ng·h/mL) | C'ₘₐₓ/AUC_{0-∞} (h⁻¹)⁻¹) | λ_{z} (h⁻¹) | Rstart (h) · | half-life (h) | HVD (h) | AUC₀₋₂₄ (ng·h/mL) | AUC_{0- 24}/AUC_{0-∞} (%) |
|---|---|---|---|---|---|---|---|---|---|---|---|---|
| 100 | 100 | Arith. mean | 20.38 | 520 | 179 | 0.0394 | 0.10 6 | 23.1 | 6.96 | 25.6 | 380 | 72.5 |
| | | SD | 6.67 | 170 | 92 | 0.0054 | 0.25 6 | 4.2 | 1.91 | 2.9 | 123 | 7.69 |
| 200 | 200 | Arith. mean | 43.13 | 1080 | 540 | 0.0395 | 0.11 1 | 25.1 | 6.69 | 26.3 | 782 | 71.3 |
| | | SD | 16.53 | 328 | 164 | 0.0079 | 0.02 9 | 4.0 | 1.84 | 5.0 | 259 | 8.8 |
| 300 | 300 | Arith. mean | 59.88 | 1641 | 587 | 0.0374 | 0.10 2 | 25.8 | 7.36 | 28.1 | 1107 | 67.9 |
| | | SD | 19.19 | 538 | 312 | 0.0092 | 0.02 9 | 3.6 | 2.21 | 6.6 | 346 | 11.0 |
| 200 | 400 | Arith. mean | 114.34 | 2866 | NC | 0.0457 | 0.09 4 | 18.7 | 8.14 | NC | 1909 | 74.6 |
| | | SD | 46.39 | 773 | NC | 0.0147 | 0.02 8 | 5.5 | 2.98 | NC | 651 | 10.9 |

| | | | | | | | | | | | | |
|---|---|---|---|---|---|---|---|---|---|---|---|---|
| NC-Not calculated | | | | | | | | | | | | |

## Claims

1. A solid dosage formulation comprising:
a core comprising a pharmacological agent dispersed in a first controlled release matrix and a coat formed over the core comprising a pharmacological agent dispersed in a second controlled release matrix,
wherein
the first controlled release matrix comprises cross-linked high amylose starch and/or the second controlled release matrix comprises a physical mixture of polyvinyl acetate and polyvinylpyrrolidone and
the initial rate of release of the agent for the second controlled release matrix is a least twice as fast as the rate of release of the agent from the first controlled release matrix when measured separately for each matrix material under USP Type I conditions with 50 mM sodium phosphate buffer and pH 6.8 and 100 rpm .

2. The formulation according to claim 1, wherein the rate of release of the agent from the coat is at least 3 times the rate of release of the agent from the core .

3. The formulation according to claim 2, wherein the rate of release of the agent from the coat is between 3 and 9 times the rate of release of the agent from the core.

4. The formulation according to any one of the preceding claims having an *in vitro* dissolution profile when measured with a USP Type I apparatus in 50 mM phosphate at a pH 6.8 and while stirring between 50 and 150 rpm as follows:
| **Elapsed Time in Hours** | **Weight-Percent Release of Agent** |
|---|---|
| 0-2 | 10-40 |
| 2-7 | 30-60 |
| 7-12 | 50-80 |
| 20 | 80-100 |

5. The formulation according to any one of the preceding claims, wherein the weight ratio of the agent in the core to the agent in the coat is in the range between 0.6 and 2.

6. The formulation according to any one of the preceding claims, wherein the agent is present in the core in an amount between 30 and 70 weight-percent of the total composition of the core.

7. The formulation according to any one of the preceding claims, wherein the weight-ratio of the matrix of the coat to the agent of the coat is in the range between 0.7 and 4.

8. The formulation according to any one of the preceding claims, wherein the polyvinyl acetate of the coat has a molecular weight in the range from 100,000 to 1,000,000.

9. The formulation according to any one of the preceding claims, wherein the polyvinylpyrrolidone of the coat has a molecular weight in the range from 10,000 to 100,000.

10. The formulation according to any one of the preceding claims, wherein the coat further comprises xanthan gum,

11. The formulation according to any one of the preceding claims, wherein the weight ratio of the core to the coat is in the range between 0.2 and 0.5.

12. The formulation according to any one of the preceding claims, wherein the first controlled release matrix comprises crosslinked high amylose starch and the second controlled release matrix comprises a physical mixture of polyvinyl acetate and polyvinylpyrrolidone.

13. The formulation according to any one of the preceding claims, wherein the agent has a solubility in water greater than 500 g/L.

14. The formulation according to any one of the preceding claims, wherein the agent has an ionizable group and the ionizable group is at least 90% ionized in gastric juices (0.1 M HCl).

15. The formulation according to any one of the preceding claims, wherein the agent of the core and of the coat is the same and is tramadol.

16. The formulation according to claim 15, wherein the formulation is a once daily oral pharmaceutical composition for controlled release of tramadol or a salt thereof, wherein the composition upon initial administration of one dose, provides a mean plasma concentration of at least 100 ng/mL within two hours of administration and continues to provide a mean plasma concentration of at least 100 ng/mL for at least 22 hours after administration.

17. The formulation according to claim 16, wherein the mean maximum plasma concentration (Cₘₐₓ) is less than 2.2 times the mean plasma concentration obtained 24 hours after administration (C₂₄ₕ).

18. The formulation according to any one of claims 15 to 17 which provides a tramadol plasma concentration which, after the time at which the maximum blood plasma concentration (Cₘₐₓ) is observed, declines in a log-linear fashion with an apparent terminal elimination rate constant (λ_{z}) of 0.12 per hour.

19. A tablet comprising the formulation according to any one of the preceding claims.

## Patentansprüche

1. Formulierung zur Verabreichung eines Feststoffs, die Folgendes umfasst:
einen Kern, der ein in einer ersten Matrix mit kontrollierter Freisetzung dispergiertes pharmakologisches Mittel umfasst, und einen über dem Kern ausgebildeten Überzug, der ein in einer zweiten Matrix mit kontrollierter Freisetzung dispergiertes pharmakologisches Mittel umfasst,
wobei
die erste Matrix mit kontrollierter Freisetzung vernetzte Stärke mit hohem Amylosegehalt umfasst und/oder die zweite Matrix mit kontrollierter Freisetzung eine physikalische Mischung von Polyvinylacetat und Polyvinylpyrrolidon umfasst, und
die anfängliche Freisetzungsgeschwindigkeit des Mittels aus der zweiten Matrix mit kontrollierter Freisetzung mindestens doppelt so schnell ist wie die Freisetzungsgeschwindigkeit des Mittels aus der ersten Matrix mit kontrollierter Freisetzung, bei getrennter Messung für jedes Matrixmaterial unter Bedingungen gemäß USP Typ I mit 50 mM Natriumphosphatpuffer, einem pH-Wert von 6,8 und 100 U/min.

2. Formulierung nach Anspruch 1, wobei die Freisetzungsgeschwindigkeit des Mittels aus dem Überzug mindestens das 3-Fache der Freisetzungsgeschwindigkeit des Mittels aus dem Kern beträgt.

3. Formulierung nach Anspruch 2, wobei die Freisetzungsgeschwindigkeit des Mittels aus dem Überzug zwischen dem 3- und 9-Fachen der Freisetzungsgeschwindigkeit des Mittels aus dem Kern beträgt.

4. Formulierung nach einem der vorhergehenden Ansprüche mit dem folgenden In-vitro-Lösungsprofil bei Messung mit einer Vorrichtung gemäß USP Typ I in 50 mM Phosphat mit einem pH-Wert von 6,8 und unter Rühren mit 50 bis 150 U/min:
| **Verstrichene Zeit in Stunden** | **Freisetzung des Mittels in Gew.-%** |
|---|---|
| 0-2 | 10-40 |
| 2-7 | 30-60 |
| 7-12 | 50-80 |
| 20 | 80-100 |

5. Formulierung nach einem der vorhergehenden Ansprüche, wobei das Gewichtsverhältnis des Mittels im Kern zu dem Mittel im Überzug im Bereich zwischen 0,6 und 2 liegt.

6. Formulierung nach einem der vorhergehenden Ansprüche, wobei das Mittel im Kern in einer Menge zwischen 30 und 70 Gew.-% der Gesamtzusammensetzung des Kerns vorliegt.

7. Formulierung nach einem der vorhergehenden Ansprüche, wobei das Gewichtsverhältnis der Matrix des Überzugs zum Mittel des Überzugs im Bereich zwischen 0,7 und 4 liegt.

8. Formulierung nach einem der vorhergehenden Ansprüche, wobei das Polyvinylacetat des Überzugs ein Molekulargewicht im Bereich von 100.000 bis 1.000.000 hat.

9. Formulierung nach einem der vorhergehenden Ansprüche, wobei das Polyvinylpyrrolidon des Überzugs ein Molekulargewicht im Bereich von 10.000 bis 100.000 hat.

10. Formulierung nach einem der vorhergehenden Ansprüche, wobei der Überzug ferner Xanthan umfasst.

11. Formulierung nach einem der vorhergehenden Ansprüche, wobei das Gewichtsverhältnis des Kerns zum Überzug im Bereich zwischen 0,2 und 0,5 liegt.

12. Formulierung nach einem der vorhergehenden Ansprüche, wobei die erste Matrix mit kontrollierter Freisetzung vernetzte Stärke mit hohem Amylosegehalt umfasst und die zweite Matrix mit kontrollierter Freisetzung eine physikalische Mischung von Polyvinylacetat und Polyvinylpyrrolidon umfasst.

13. Formulierung nach einem der vorhergehenden Ansprüche, wobei das Mittel eine Löslichkeit in Wasser größer als 500 g/L hat.

14. Formulierung nach einem der vorhergehenden Ansprüche, wobei das Mittel eine ionisierbare Gruppe hat und die ionisierbare Gruppe in Magensäften (0,1 M HCl) zu mindestens 90% ionisiert ist.

15. Formulierung nach einem der vorhergehenden Ansprüche, wobei das Mittel des Kerns und des Überzugs identisch ist und Tramadol ist.

16. Formulierung nach Anspruch 15, wobei die Formulierung eine einmal täglich oral zu verabreichende pharmazeutische Zusammensetzung zur kontrollierten Freisetzung von Tramadol oder einem Salz davon ist, wobei die Zusammensetzung nach erstmaliger Verabreichung einer Dosis eine mittlere Plasmakonzentration von mindestens 100 ng/ml innerhalb von zwei Stunden nach Verabreichung bereitstellt und weiterhin für mindestens 22 Stunden nach Verabreichung eine mittlere Plasmakonzentration von mindestens 100 ng/ml bereitstellt.

17. Formulierung nach Anspruch 16, wobei die mittlere maximale Plasmakonzentration (Cₘₐₓ) weniger als das 2,2-Fache der 24 Stunden nach Verabreichung erhaltenen mittleren Plasmakonzentration (C₂₄ₕ) beträgt.

18. Formulierung nach einem der Ansprüche 15 bis 17, die eine Plasmakonzentration von Tramadol bereitstellt, die nach dem Zeitpunkt, wo die maximale Blutplasmakonzentration (Cₘₐₓ) festgestellt wird, mit einer Konstante (λ_{z}) der scheinbaren Eliminierungsendgeschwindigkeit von 0,12 pro Stunde linearlogarithmisch abfällt.

19. Tablette mit der Formulierung nach einem der vorhergehenden Ansprüche.

## Revendications

1. Formulation posologique solide comprenant :
un noyau, comprenant un agent pharmaceutique dispersé dans une première matrice pour libération retardée, et un enrobage, formé par-dessus le noyau et comprenant un agent pharmaceutique dispersé dans une deuxième matrice pour libération retardée,
dans laquelle formulation :
- la première matrice pour libération retardée comprend un amidon réticulé à taux élevé d'amylose et/ou la deuxième matrice pour libération retardée comprend un mélange physique de poly(acétate de vinyle) et de poly(vinyl-pyrrolidone),
- et la vitesse initiale de libération de l'agent de la deuxième matrice pour libération retardée vaut au moins le double de la vitesse de libération de l'agent de la première matrice pour libération retardée, ces vitesses étant mesurées séparément pour le matériau de chaque matrice dans des conditions USP Type 1, dans une solution tampon à 50 mM de phosphate de sodium, à un pH de 6,8 et sous agitation à 100 t/min.

2. Formulation conforme à la revendication 1, pour laquelle la vitesse de libération de l'agent de l'enrobage vaut au moins 3 fois la vitesse de libération de l'agent du noyau.

3. Formulation conforme à la revendication 2, pour laquelle la vitesse de libération de l'agent de l'enrobage vaut entre 3 et 9 fois la vitesse de libération de l'agent du noyau.

4. Formulation conforme à l'une des revendications précédentes, dont le profil de dissolution *in vitro,* mesuré avec un appareil USP Type 1, dans une solution tampon à 50 mM de phosphate de sodium, à un pH de 6,8 et sous agitation à une vitesse de 50 à 150 t/min, est le suivant :
| Temps écoulé, en heures | Quantité d'agent libérée, en pourcentage pondéral |
|---|---|
| 0-2 | 10-40 |
| 2-7 | 30-60 |
| 7-12 | 50-80 |
| 20 | 80 - 100 |

5. Formulation conforme à l'une des revendications précédentes, dans laquelle le rapport pondéral de l'agent présent dans le noyau à l'agent présent dans l'enrobage se situe dans l'intervalle allant de 0,6 à 2.

6. Formulation conforme à l'une des revendications précédentes, dans laquelle l'agent présent dans le noyau s'y trouve en une quantité qui représente de 30 à 70 % du poids total de la composition du noyau.

7. Formulation conforme à l'une des revendications précédentes, dans laquelle le rapport pondéral de la matrice de l'enrobage à l'agent présent dans l'enrobage se situe dans l'intervalle allant de 0,7 à 4.

8. Formulation conforme à l'une des revendications précédentes, dans laquelle le poly(acétate de vinyle) de l'enrobage présente une masse molaire située dans l'intervalle allant de 100 000 à 1 000 000.

9. Formulation conforme à l'une des revendications précédentes, dans laquelle la poly(vinyl-pyrrolidone) de l'enrobage présente une masse molaire située dans l'intervalle allant de 10 000 à 100 000.

10. Formulation conforme à l'une des revendications précédentes, dans laquelle l'enrobage comprend en outre de la gomme xanthane.

11. Formulation conforme à l'une des revendications précédentes, dans laquelle le rapport pondéral du noyau à l'enrobage se situe dans l'intervalle allant de 0,2 à 0,5.

12. Formulation conforme à l'une des revendications précédentes, dans laquelle la première matrice pour libération retardée comprend un amidon réticulé à taux élevé d'amylose et la deuxième matrice pour libération retardée comprend un mélange physique de poly(acétate de vinyle) et de poly(vinyl-pyrrolidone),

13. Formulation conforme à l'une des revendications précédentes, dans laquelle la solubilité de l'agent dans l'eau est supérieure à 500 g/L.

14. Formulation conforme à l'une des revendications précédentes, dans laquelle l'agent comporte un groupe ionisable, lequel groupe ionisable est ionisé à au moins 90 % dans du suc gastrique (à 0,1 M de HCl).

15. Formulation conforme à l'une des revendications précédentes, dans laquelle l'agent est le même dans le noyau et dans l'enrobage, et cet agent est du tramadol.

16. Formulation conforme à la revendication 15, laquelle formulation est une composition pharmaceutique à prendre par voie orale une fois par jour, conçue pour libérer avec retard du tramadol ou l'un de ses sels, laquelle composition, suite à la prise d'une dose, donne une concentration plasmatique moyenne d'au moins 100 ng/mL dans les deux heures suivant la prise et continue à donner une concentration plasmatique moyenne d'au moins 100 ng/mL pendant au moins 22 heures après la prise.

17. Formulation conforme à la revendication 16, avec laquelle la concentration plasmatique maximale moyenne (Cₘₐₓ) vaut moins de 2,2 fois la concentration plasmatique moyenne obtenue 24 heures après la prise (C₂₄ₕ).

18. Formulation conforme à l'une des revendications 15 à 17, qui donne une concentration plasmatique de tramadol qui, à partir de l'instant où l'on observe la concentration plasmatique maximale (Cₘₐₓ), décroît en mode log-linéaire, avec une constante apparente de vitesse terminale d'élimination (λ_{z}) de 0,12 h⁻¹.

19. Comprimé comprenant une formulation conforme à l'une des revendications précédentes.
